# EUROPÄISCHE PATENTSCHRIFT

(11) **EP 0 869 959 B1**
(45) Veröffentlichungstag und Bekanntmachung des Hinweises auf die Patenterteilung: **17.05.2000**
(21) Anmeldenummer: 96943081.8
(22) Anmeldetag: 11.12.1996
(51) Int. Cl.: C07D 493/06, C09B 3/14, C09B 5/62

(54) **1,7-DISUBSTITUIERTE PERYLEN-3,4,9,10-TETRACARBONSÄUREN, DEREN DIANHYDRIDE UND DIIMIDE**
1,7-DISUBSTITUTED PERYLENE-3,4,9,10-TETRACARBOXYLIC ACIDS AND DIANHYDRIDES AND DIIMIDES OF SAID ACIDS
ACIDES 1,7-DISUBSTITUES PERYLENE-3,4,9,10-TETRACARBOXYLIQUES, LEURS DIANHYDRIDES ET LEURS DIIMIDES

(30) Priorität: 18.12.1995 DE 19547210
(43) Veröffentlichungstag der Anmeldung: 14.10.1998
(73) Patentinhaber: BASF AKTIENGESELLSCHAFT, 67056 Ludwigshafen (DE)
(72) Erfinder: BÖHM, Arno, D-68305 Mannheim (DE); ARMS, Harald, D-67551 Worms (DE); HENNING, Georg, D-67061 Ludwigshafen (DE); BLASCHKA, Peter, D-67069 Ludwigshafen (DE)
(86) Internationale Anmeldenummer: EP9605525
(87) Internationale Veröffentlichungsnummer: WO9722608

(56) Entgegenhaltungen:
- GB-A- 967 178
- US-A- 4 496 731
- US-A- 4 667 036
- US-A- 5 248 774
- CHEMICAL ABSTRACTS, vol. 110, no. 3, 16.Januar 1989 Columbus, Ohio, US; abstract no. 23496v, V. I. ROGOVIK ET. AL.: "Chemistry of Perylene. Halo derivatives of 3,4,9,10-perylene tetracarboxylic acid" Seite 513; Spalte 2; XP002028674 & ZH. ORG. KHIM., Bd. 24, Nr. 3, 1988, Seiten 635-9,
- JOURNAL OF ORGANIC CHEMISTRY OF THE USSR ROGOVIK V. I. AND GUTNIK L.F.: 'Chemistry of Perylene. Halogen Derivatives of Perylene-3,4,9,10-Tetracarboxylic Acid' Bd. 24, Nr. 3, 1988, Plenum Publishing Corporation, Seiten 569-573. Translated from ZHURNAL ORGANICHESKOI KHIMII, Bd 24, Nr, 3 Seiten 635-639, März 1988
- DYES AND PIGMENTS SEYBOLD, G. AND WAGENBLAST, G.: 'New Perylene and Violanthrone Dyestuffs for Fluorescent Collectors' Bd. 11, Nr. 4, BARKING, ESSEX, GB, Seiten 303 - 317, XP000084462

## Beschreibung

Die vorliegende Erfindung betrifft neue, 1,7-disubstituierte Perylen-3,4,9,10-tetracarbonsäuredianhydride der allgemeinen Formel I und Perylen-3,4,9,10-tetracarbonsäuren der allgemeinen Formel Ia 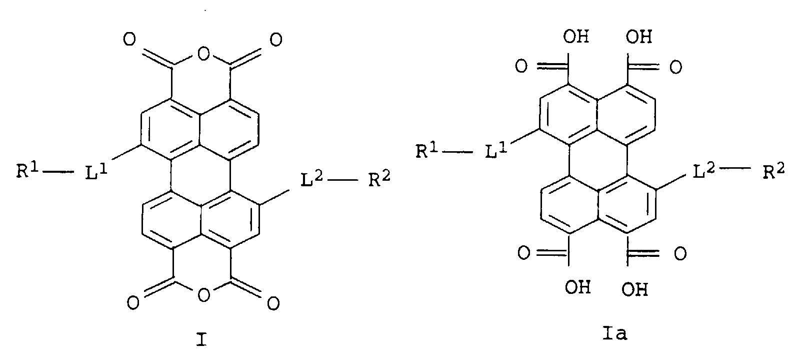 in der die Variablen folgende Bedeutung haben:
- L¹, L²: unabhängig voneinander 1,2-Ethylen, 1,2-Ethenylen und 1,2-Ethinylen;
- R¹, R²: unabhängig voneinander Wasserstoff oder C₁-C₃₀-Alkyl, dessen Kohlenstoffkette durch eine oder mehrere Gruppierungen -O-, -S-, -NR³-, -CO- und/oder -SO₂- unterbrochen sein kann und/oder das durch -COOR³, -SO₃R³, Hydroxy, Cyano, C₁-C₆-Alkoxy, C₅-C₈-Cycloalkyl, Aryl oder einen über ein Stickstoffatom gebundenen, 5- bis 7-gliedrigen heterocyclischen Rest, der weitere Heteroatome enthalten kann und/oder aromatisch sein kann, ein- oder mehrfach substituiert sein kann, wobei R³ für Wasserstoff oder C₁-C₆-Alkyl steht,
sowie ein Verfahren zur Herstellung der Perylen-3,4,9,10-tetracarbonsäuredianhydride (I) bzw. der Säuren (Ia) und deren Verwendung als Pigmente, Laserfarbstoffe und Vorstufen für die Herstellung von Fluoreszenzfarbstoffen, polymeren Farbmitteln, Pigmenten und Pigmentadditiven.

Weiterhin betrifft die Erfindung neue, 1,7-disubstituierte Perylen-3,4,9,10-tetracarbonsäurediimide der allgemeinen Formel VI 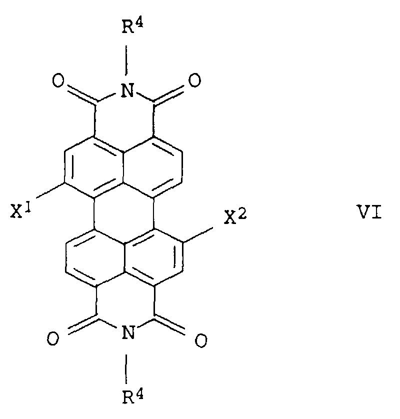 in der die Variablen folgende Bedeutung haben:
- X¹: Brom oder einen Rest der Formel -L-R, wobei
L für 1,2-Ethylen, 1,2-Ethenylen oder 1,2-Ethinylen steht und
R Wasserstoff oder C₁-C₃₀-Alkyl bedeutet, dessen Kohlenstoffkette durch eine oder mehrere Gruppierungen -O-, -S-, -NR³-, -CO- und/oder -SO₂- unterbrochen sein kann und/oder das durch -COOR³, -SO₃R³, Hydroxy, Cyano, C₁-C₆-Alkoxy, C₅-C₈-Cycloalkyl, Aryl oder einen über ein Stickstoffatom gebundenen, 5- bis 7-gliedrigen heterocyclischen Rest, der weitere Heteroatome enthalten kann und/oder aromatisch sein kann, ein- oder mehrfach substituiert sein kann, wobei R³ für Wasserstoff oder C₁-C₆-Alkyl steht;
- X²: Brom oder einen Rest der Formel -L-R;
- R⁴: C₄-C₃₀-Alkyl, dessen Kohlenstoffkette durch eine oder mehrere Gruppierungen -O-, -S- oder -CO- unterbrochen sein kann, C₅-C₈-Cycloalkyl oder Aryl, das durch C₁-C₆-Alkyl oder C₁-C₆-Alkoxy ein- oder mehrfach substituiert sein kann,
als Zwischenprodukte für die Perylen-3,4,9,10-tetracarbonsäuredianhydride (I) bzw. die Säuren (Ia) sowie ein Verfahren zur Herstellung der Perylen-3,4,9,10-tetracarbonsäurediimide (VI).

Perylen-3,4,9,10-tetracarbonsäuren bzw. deren Anhydride stellen bekanntermaßen wichtige Zwischenprodukte für die Herstellung von Perylimidpigmenten und -farbstoffen dar (z.B. US-A-4 496 731 und GB-A-967 178), eignen sich aber auch selbst zur Einfärbung bzw. Pigmentierung von hochmolekularen organischen Materialien.

Neben der unsubstituierten Perylen-3,4,9,10-tetracarbonsäure, die durch Verseifung von Perylen-3,4,9,10-tetracarbonsäurediimid in konzentrierter Schwefelsäure bei Temperaturen um 200°C erhältlich ist, sind vor allem auch im Perylengerüst substituierte Perylentetracarbonsäuren von Interesse, deren Anwendungseigenschaften, wie Löslichkeit, Eigenfarbe und Fluoreszenz, durch Einführung geeigneter Substituenten gezielt beeinflußt werden können.

Aus der WO-A-94/25504 sind 1,6,7,12-tetraaroxysubstituierte Perylen-3,4,9,10-tetracarbonsäuredianhydride bekannt, die durch alkalische Verseifung der entsprechenden Diimide in einem polaren, protischen Lösungsmittel hergestellt werden. Die tetraaroxysubstituierten Diimide selbst wurden durch Umsetzung der tetrachlorierten Diimide mit Arylaten erhalten (EP-A-227 980).

1,7-Disubstituierte Perylen-3,4,9,10-tetracarbonsäuren wie die erfindungsgemäßen Verbindungen (Ia), die wie alle Perylen-3,4,9,10-tetracarbonsäuren in der Regel in Form der Dianhydride vorliegen, sind bislang nicht bekannt. Auch bei den in der EP-A-39 912, der DE-A-412 122 und den US-A-4 667 036 und 5 248 774 beschriebenen dihalogenierten Perylen-3,4,9,10-tetracarbonsäurediimiden handelt es sich stets um Gemische von verschieden stark halogenierten Produkten (insbesondere von tetra-, tri- und monohalogenierten Produkten), die dihalogenierten Diimide konnten nicht gezielt hergestellt werden. Dies gilt ebenso für die in Zh. Org. Khim., 24, 635 - 639 (1988; C.A. 110 : 23496v) beschriebenen dihalogenierten Perylen-3,4,9,10-tetracarbonsäuredianhydride.

Der Erfindung lag daher die Aufgabe zugrunde, neue, 1,7-disubstituierte Perylen-3,4,9,10-tetracarbonsäuren bzw. -dianhydride bereitzustellen.

Demgemäß wurden die 1,7-disubstituierten Perylen-3,4,9,10-tetracarbonsäuredianhydride und die entsprechenden Säuren der eingangs definierten Formeln I und Ia (im folgenden als "Dianhydride I" bezeichnet) gefunden, die symmetrisch oder unsymmetrisch substituiert sein können.

Bevorzugte Dianhydride I sind dem Unteranspruch zu entnehmen.

Weiterhin wurde ein Verfahren zur Herstellung der symmetrischen Dianhydride I gefunden, welches dadurch gekennzeichnet ist, daß man
a) 1,7-Dibromperylen-3,4,9,10-tetracarbonsäuredianhydrid (II) bzw. 1,7-Dibromperylen-3,4,9,10-tetracarbonsäure (IIa) in Gegenwart eines polaren aprotischen Lösungsmittels und gegebenenfalls eines Imidierungskatalysators mit einem primären Amin der allgemeinen Formel III

   R⁴ - NH₂ III

   in der R⁴ C₄-C₃₀-Alkyl, dessen Kohlenstoffkette durch eine oder mehrere Gruppierungen -O-, -S- oder -CO- unterbrochen sein kann, C₅-C₈-Cycloalkyl oder Aryl, das durch C₁-C₆-Alkyl oder C₁-C₆-Alkoxy ein- oder mehrfach substituiert sein kann, bedeutet, umsetzt,
b) die in Schritt a) gebildeten 1,7-Dibromperylen-3,4,9,10-tetracarbonsäurediimide der allgemeinen Formel IV 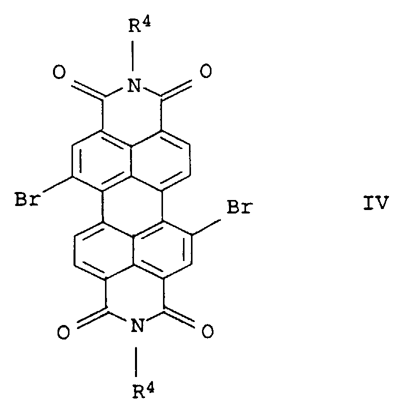 in Gegenwart eines aprotischen Lösungsmittels, eines Palladiumkomplexes als Katalysator, eines Kupfersalzes als Cokatalysator und einer Base mit einem 1-Alkin der allgemeinen Formel V

   H - C ≡ C - R¹ V

   in einem Molverhältnis von 1:2 bis 1:4 umsetzt und
c) die in Schritt b) gebildeten symmetrischen, 1,7-disubstituierten Perylen-3,4,9,10-tetracarbonsäurediimide der allgemeinen Formel VI' 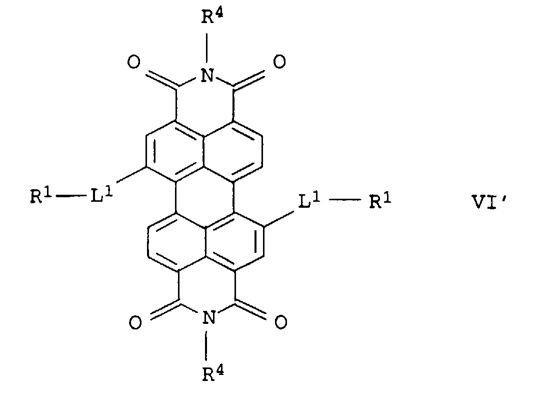 gewünschtenfalls nach zusätzlicher Reduktion der ungesättigten Bindungen in L¹ in Gegenwart eines polaren protischen Lösungsmittels und einer Base zu den symmetrischen Dianhydriden I verseift.

Außerdem wurde ein Verfahren zur Herstellung der unsymmetrischen Dianhydride I gefunden, welches dem Verfahren zur Herstellung der symmetrischen Dianhydride I in Schritt a) entspricht und bei welchem man die 1,7-Dibromperylen-3,4,9,10-tetracarbonsäurediimide IV jedoch im ebenfalls in Gegenwart eines aprotischen Lösungsmittels, eines Palladiumkomplexes als Katalysator, eines Kupfersalzes als Cokatalysator und einer Base vorgenommenen Schritt b) zunächst mit einem 1-Alkin der allgemeinen Formel Va

H - C ≡ C - R¹ Va

und anschließend mit einem anderen 1-Alkin der allgemeinen Formel Vb

H - C ≡ C - R² Vb

jeweils in einem Molverhältnis von 1:1 bis 1:2 umsetzt und die gebildeten unsymmetrischen, 1,7-disubstituierten Perylen-3,4,9,10-tetracarbonsäurediimide der allgemeinen Formel VI" 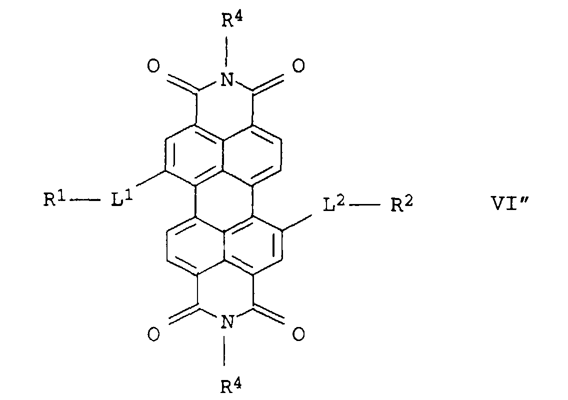 dann in Schritt c) gewünschtenfalls nach zusätzlicher Reduktion der ungesättigten Bindungen in L¹ und L² in Gegenwart eines polaren protischen Lösungsmittels und einer Base zu den unsymmetrischen Dianhydriden I verseift.

Zudem wurden die 1,7-disubstituierten Perylen-3,4,9,10-tetracarbonsäurediimide der eingangs definierten Formel VI (im folgenden als "Perylimide VI" bezeichnet), die ebenfalls symmetrisch oder unsymmetrisch substituiert sein können, als Zwischenprodukte für die Dianhydride I sowie Verfahren zur Herstellung der Perylimide VI gefunden, welche die Schritte a) und b) des Verfahrens zur Herstellung der entsprechenden Dianhydride I umfassen.

Bevorzugte Perylimide VI sind dem Unteranspruch zu entnehmen.

Nicht zuletzt wurde die Verwendung der Dianhydride I als Pigmente, Laserfarbstoffe und Vorstufen für die Herstellung von Fluoreszenzfarbstoffen, polymeren Farbmitteln, Pigmenten und Pigmentadditiven gefunden.

Schließlich wurde auch die Verwendung der Perylimide VI als Pigmente und Farbstoffe zum Einfärben von hochmolekularen organischen und von anorganischen Materialien, als Laserfarbstoffe und organische Materialien für Elektrolumineszenzanwendungen gefunden.

Alle in den Formeln I (auch Ia), III, IV, V (auch Va und Vb) und VI (auch VI' und VI") auftretenden Alkylgruppen können sowohl geradkettig als auch verzweigt sein. Aromatische Reste, die substituiert sind, können im allgemeinen bis zu 3, bevorzugt 1 oder 2 der genannten Substituenten aufweisen.

Als Beispiele für geeignete Reste R¹ sowie R² (bzw. für deren Substituenten) seien neben Wasserstoff im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436);
2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 4,7-Dithiaoctyl, 4,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-(N-Methylamino)- und 2-(N-Ethylamino)ethyl, 2-(N,N-Dimethylamino)ethyl, 2- und 3-(N,N-Dimethylamino)propyl, 3-(N-Isopropylamino)propyl, 2- und 4-(N-Propylamino)butyl, 2- und 4-(N,N-Dimethylamino)butyl, 6-Methyl-3,6-diazaheptyl, 3,6-Dimethyl-3,6-diazaheptyl, 3,6-Diazoaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
Propan-2-on-1-yl, Butan-3-on-1-yl, Butan-3-on-2-yl und 2-Ethylpentan-3-on-1-yl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Methylcarboxymethyl, Ethylcarboxymethyl, Propylcarboxymethyl, Butylcarboxymethyl, Pentylcarboxymethyl, Hexylcarboxymethyl, Methyl-2-carboxyethyl, Ethyl-2-carboxyethyl, Propyl-2-carboxyethyl, Butyl-2-carboxyethyl, Pentyl-2-carboxyethyl, Hexyl-2-carboxyethyl, Methyl-3-carboxypropyl, Ethyl-3-carboxypropyl, Propyl-3-carboxypropyl, Butyl-3-carboxypropyl, Pentyl-3-carboxypropyl, Hexyl-3-carboxypropyl, Methyl-4-carboxybutyl, Methyl-5-carboxypentyl, Methyl-6-carboxyhexyl, Methyl-8-carboxyoctyl, Methyl-10-carboxydecyl, Methyl-12-carboxydodecyl und Methyl-14-carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
Methylsulfomethyl, Ethylsulfomethyl, Propylsulfomethyl, Butylsulfomethyl, Pentylsulfomethyl, Hexylsulfomethyl, Methyl-2-sulfoethyl, Ethyl-2-sulfoethyl, Propyl-2-sulfoethyl, Butyl-2-sulfoethyl, Pentyl-2-sulfoethyl, Hexyl-2-sulfoethyl, Methyl-3-sulfopropyl, Ethyl-3-sulfopropyl, Propyl-3-sulfopropyl, Butyl-3-sulfopropyl, Pentyl-3-sulfopropyl und Hexyl-3-sulfopropyl, Methyl-4-sulfobutyl, Methyl-5-sulfopentyl, Methyl-6-sulfohexyl, Methyl-8-sulfooctyl, Methyl-10-sulfodecyl, Methyl-12-sulfododecyl und Methyl-14-sulfotetradecyl;
2-Hydroxyethyl, 2- und 3-Hydroxypropyl, 1-Hydroxyprop-2-yl, 2- und 4-Hydroxybutyl, 1-Hydroxybut-2-yl und 8-Hydroxy-4-oxaoctyl, 2-Cyanoethyl, 3-Cyanopropyl, 2-Methyl-3-ethyl-3-cyanopropyl, 7-Cyano-7-ethylheptyl und 4-Methyl-7-methyl-7-cyanoheptyl; Methoxy, Ethoxy, Propoxy, Isopropoxy, Butoxy, Isobutoxy, tert.-Butoxy, Pentoxy, Isopentoxy, Neopentoxy, tert.-Pentoxy und Hexoxy;
Cyclopentyl, Cyclohexyl, Cycloheptyl, Cyclooctyl, 2-Dioxanyl, 4-Morpholinyl, 2- und 3-Tetrahydrofuryl, 1-, 2- und 3-Pyrrolidinyl und 1-, 2-, 3- und 4-Piperidyl;
Phenyl, 2-Naphthyl, 2- und 3-Pyrryl, 2-, 3- und 4-Pyridyl, 2-, 4- und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl.

Als Beispiele für besonders bevorzugte Reste -L-R (auch -L¹-R¹ und -L²-R²) sind unsubstituiertes oder durch C₁-C₁₈-Alkyl, vor allem C₄-C₈-Alkyl, das insbesondere endständig (ω-Position) durch Cyano, Hydroxy, Carboxy, Methyl- oder Ethylcarboxy substituiert sein kann, substituiertes Ethenyl und Ethinyl zu nennen. Im einzelnen seien genannt:
Ethinyl, 1-Propinyl, 1-Butinyl, 1-Pentinyl, 3-Methyl-1-butinyl, 1-Hexinyl, 3- und 4-Methyl-1-pentinyl, 3,3-Dimethyl-1-butinyl, 1-Heptinyl, 3-, 4- und 5-Methyl-1-hexinyl, 3,3-, 3,4- und 4,4-Dimethyl-l-pentinyl, 3-Ethyl-1-pentinyl, 1-Octinyl, 3-, 4-, 5- und 6-Methyl-1-heptinyl, 3,3-, 3,4-, 3,5-, 4,4- und 4,5-Dimethyl-l-hexinyl, 3-, 4- und 5-Ethyl-1-hexinyl, 3-Ethyl-3-methyl-1-pentinyl, 3-Ethyl-4-methyl-l-pentinyl, 3,3,4- und 3,4,4-Trimethyl-1-pentinyl, 1-Noninyl, 1-Decinyl, 1-Undecinyl und 1-Dodecinyl;
4-Cyano-1-butinyl, 5-Cyano-1-pentinyl, 6-Cyano-1-hexinyl, 7-Cyano-1-heptinyl und 8-Cyano-1-octinyl;
4-Hydroxy-l-butinyl, 5-Hydroxy-1-pentinyl, 6-Hydroxy-1-hexinyl, 7-Hydroxy-l-heptinyl, 8-Hydroxy-1-octinyl, 9-Hydroxy-1-noninyl, 10-Hydroxy-l-decinyl, 11-Hydroxy-1-undecinyl und 12-Hydroxy-1-dodecinyl;
4-Carboxy-1-butinyl, 5-Carboxy-l-pentinyl, 6-Carboxy-1-hexinyl, 7-Carboxy-1-heptinyl, 8-Carboxy-1-octinyl, 4-Methylcarboxy-1-butinyl, 5-Methylcarboxy-1-pentinyl, 6-Methylcarboxy-1-hexinyl, 7-Methylcarboxy-1-heptinyl, 8-Methylcarboxy-1-octinyl, 4-Ethylcarboxy-1-butinyl, 5-Ethylcarboxy-1-pentinyl, 6-Ethylcarboxy-1-hexinyl, 7-Ethylcarboxy-1-heptinyl und 8-Ethylcarboxy-1-octinyl;
Ethenyl, 1-Propenyl, 1-Butenyl, 1-Pentenyl, 3-Methyl-1-butenyl, 1-Hexenyl, 3- und 4-Methyl-1-pentenyl, 3,3-Dimethyl-1-butenyl, 1-Heptenyl, 3-, 4- und 5-Methyl-1-hexenyl, 3,3-, 3,4- und 4,4-Dimethyl-1-pentenyl, 3-Ethyl-1-pentenyl, 1-Octenyl, 3-, 4-, 5- und 6-Methyl-1-heptenyl, 3,3-, 3,4-, 3,5-, 4,4- und 4,5-Dimethyl-1-hexenyl, 3-, 4-, und 5-Ethyl-1-hexenyl, 3-Ethyl-3-methyl-1-pentenyl, 3-Ethyl-4-methyl-1-pentenyl, 3,3,4- und 3,4,4-Trimethyl-1-pentenyl, 1-Nonenyl, 1-Decenyl, 1-Undecenyl und 1-Dodecenyl;
4-Cyano-1-butenyl, 5-Cyano-1-pentenyl, 6-Cyano-1-hexenyl, 7-Cyano-1-heptenyl und 8-Cyano-1-octenyl;
4-Hydroxy-1-butenyl, 5-Hydroxy-1-pentenyl, 6-Hydroxy-1-hexenyl, 7-Hydroxy-1-heptenyl, 8-Hydroxy-1-octenyl, 9-Hydroxy-1-nonenyl, 10-Hydroxy-1-decenyl, 11-Hydroxy-1-undecenyl und 12-Hydroxy-1-dodecenyl;
4-Carboxy-l-butenyl, 5-Carboxy-1-pentenyl, 6-Carboxy-1-hexenyl, 7-Carboxy-1-heptenyl, 8-Carboxy-1-octenyl, 4-Methylcarboxy-1-butenyl, 5-Methylcarboxy-1-pentenyl, 6-Methylcarboxy-1-hexenyl, 7-Methylcarboxy-1-heptenyl, 8-Methylcarboxy-1-octenyl, 4-Ethylcarboxy-1-butenyl, 5-Ethylcarboxy-1-pentenyl, 6-Ethylcarboxy-1-hexenyl, 7-Ethylcarboxy-1-heptenyl und 8-Ethylcarboxy-1-octenyl.

Beispiele für geeignete Reste R³ sind neben Wasserstoff die obengenannten C₁-C₆-Alkylreste.

Als Beispiele für geeignete Reste R⁴ sind die oben aufgeführten C₄-C₃₀-Alkylreste, die durch -O-, -S- oder -CO- unterbrochenen C₄-C₃₀-Alkylreste und die C₅-C₈-Cycloalkylreste sowie Arylreste, z.B. Naphthyl und insbesondere Phenyl, zu nennen, die durch 1, 2 oder 3 der genannten C₁-C₆-Alkyl- oder -Alkoxyreste substituiert sein können, wie
2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3- und 4-Butylphenyl, 2,4-, 3,5- und 2,6-Dibutylphenyl, 2,4,6-Tributylphenyl, 2-, 3- und 4-Isobutylphenyl, 2,4-, 3,5 und 2,6-Diisobutylphenyl, 2,4,6-Triisobutylphenyl; 2-, 3- und 4-Methoxyphenyl, 2,4-, 3,5- und 2,6-Dimethoxyphenyl, 2,4,6-Trimethoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3- und 4-Isopropoxyphenyl, 2,4- und 2,6-Diisopropoxyphenyl und 2-, 3- und 4-Butoxyphenyl.

Die erfindungsgemäße Herstellung der Dianhydride I erfolgt in mehreren Stufen. In Schritt a) wird 1,7-Dibromperylen-3,4,9,10-tetracarbonsäuredianhydrid (II) mit einem primären Amin III zu dem entsprechenden 1,7-Dibromperylen-3,4,9,10-tetracarbonsäurediimid IV umgesetzt, welches in Schritt b) mit einem 1-Alkin V (oder stufenweise mit verschiedenen 1-Alkinen Va und Vb) zum Perylimid VI umgesetzt wird, das schließlich in Schritt c), gewünschtenfalls nach zusätzlicher Reduktion der ungesättigten Bindung im Brückenglied L, basisch zum Dianhydrid I verseift wird.

Das als Ausgangsprodukt für das erfindungsgemäße Herstellungsverfahren dienende 1,7-Dibromperylen-3,4,9,10-tetracarbonsäuredianhydrid (II) kann durch selektive Bromierung von Perylen-3,4,9,10-tetracarbonsäuredianhydrid in 100 gew.-%iger Schwefelsäure (Monohydrat) erhalten werden.

Zweckmäßigerweise geht man dabei so vor, daß man Perylen-3,4,9,10-tetracarbonsäuredianhydrid zunächst 2 bis 16 h in der Schwefelsäure rührt und diese Mischung anschließend nach Zugabe eines Halogenierungskatalysators wie Iod (bevorzugt 30 bis 40 mmol je mol Anhydrid) auf die Reaktionstemperatur (in der Regel 80 bis 90°C) erhitzt. Dann tropft man das Brom langsam (üblicherweise in 6 bis 10 h) zu, wobei bevorzugt 2 bis 2,5 mol Brom (Br₂) je mol Anhydrid verwendet werden. Nach Abkühlen auf Raumtemperatur und Verdrängen des nicht umgesetzten Broms durch Stickstoff erniedrigt man die Schwefelsäurekonzentration durch portionsweise Zugabe von Wasser auf etwa 85 bis 88 Gew.-%.

Die Aufarbeitung des Reaktionsgemisches auf das 1,7-Dibromperylen-3,4,9,10-tetracarbonsäuredianhydrid (II) kann dann durch Abfiltrieren des ausgefallenen Produkts, Waschen mit 85 bis 88 gew.-%iger Schwefelsäure, Einrühren in Wasser, erneutes Abfiltrieren, Waschen mit Wasser und anschließendes Trocknen erfolgen.

Schritt a) des erfindungsgemäßen Herstellungsverfahrens, die Umsetzung des 1,7-Dibromperylen-3,4,9,10-tetracarbonsäuredianhydrids (II) mit dem primären Amin, wird in Gegenwart eines polaren aprotischen Lösungsmittels und gegebenenfalls eines Imidierungskatalysators vorgenommen.

Als polare aprotische Lösungsmittel eignen sich dabei vor allem aprotische Stickstoffheterocyclen wie Pyridin, Pyrimidin, Chinolin, Isochinolin, Chinaldin, N-Methylpiperidin, N-Methylpiperidon und insbesondere N-Methylpyrrolidon.

Die Menge an Lösungsmittel ist an sich nicht kritisch, üblicherweise kommen 5 bis 20 kg, vorzugsweise 10 bis 15 kg, Lösungsmittel je kg (II) zum Einsatz.

Als Imidierungskatalysatoren eignen sich organische und anorganische Säuren, z.B. Ameisensäure, Essigsäure, Propionsäure und Phosphorsäure, die bevorzugt in möglichst konzentrierter Form eingesetzt werden, sowie organische und anorganische Salze von Übergangsmetallen wie Zink, Eisen und Kupfer und von Magnesium, z.B. Zinkacetat, Zinkpropionat, Zinkoxid, Eisen(II)acetat, Eisen(III)chlorid, Eisen(II)sulfat, Kupfer(II)acetat, Kupfer(II)oxid und Magnesiumacetat. Selbstverständlich kann man auch Mischungen der genannten Katalysatoren verwenden.

Die Anwesenheit eines Imidierungskatalysators empfiehlt sich insbesondere bei der Umsetzung aromatischer Amine und ist auch bei der Umsetzung cycloaliphatischer Amine vorteilhaft, während bei der Umsetzung vor allem kurzkettiger aliphatischer Amine üblicherweise kein Katalysator erforderlich ist.

In der Regel werden 5 bis 80 Gew.-% Katalysator, bezogen auf (II), eingesetzt. Bevorzugte Mengen betragen bei den organischen Säuren 50 bis 80 Gew.-% und bei den Übergangsmetall- und Magnesiumsalzen 10 bis 40 Gew.-%, jeweils bezogen auf (II).

Als primäre Amine können bei dem erfindungsgemäßen Herstellungsverfahren alle unter den Reaktionsbedingungen stabilen Amine eingesetzt werden, die mit Perylen-3,4,9,10-tetracarbonsäuredianhydriden Diimide bilden, die unter basischen Bedingungen verseift werden können.

Beispiele für besonders bevorzugte primäre Amine III sind Stearylamin, 5-Nonylamin, Cyclopentylamin, Cyclohexylamin, Cycloheptylamin, Anilin, 4-Methylanilin, 4-Ethylanilin, 4-tert.-Butylanilin, 3,5-Dimethylanilin, 3,5-Diethylanilin und 3,5-Di-tert.-butylanilin.

Üblicherweise liegt das Molverhältnis von Amin III zu (II) bei etwa 2:1 bis 4:1, vorzugsweise bei etwa 2,2:1 bis 3:1.

Die Reaktionstemperatur beträgt in Schritt a) im allgemeinen 40 bis 180°C. Bei der Umsetzung aliphatischer und cycloaliphatischer Amine sind Temperaturen von 60 bis 100°C und bei der Umsetzung aromatischer Amine Temperaturen von 120 bis 160°C bevorzugt.

Es empfiehlt sich, unter Verwendung einer Schutzgasatmosphäre (z.B. Stickstoff) zu arbeiten.

Man kann Schritt a) des erfindungsgemäßen Verfahrens bei Normaldruck oder bei einem Überdruck von üblicherweise bis zu 10 bar durchführen. Die Arbeitsweise unter Druck ist vor allem beim Einsatz flüchtiger Amine (Siedepunkt ≤ etwa 180°C) zweckmäßig.

Üblicherweise ist die Umsetzung in 2 bis 10 h, vor allem in 4 bis 7 h, beendet.

Verfahrenstechnisch geht man in Schritt a) zweckmäßigerweise wie folgt vor:

Man legt 1,7-Dibromperylen-3,4,9,10-tetracarbonsäuredianhydrid (II), Lösungsmittel und gegebenenfalls Katalysator vor, fügt das Amin III unter Rühren bei Raumtemperatur zu, spült die Apparatur etwa 15 min mit Stickstoff, erhitzt das Gemisch unter Rühren auf die Reaktionstemperatur und hält es etwa 4 bis 7 h bei dieser Temperatur. Nach dem Abkühlen auf Raumtemperatur wird das Reaktionsprodukt abfiltriert, mit einem aliphatischen Alkohol wie Methanol gewaschen und getrocknet.

Soll die Umsetzung unter Druck vorgenommen werden, so verwendet man eine Druckapparatur als Reaktionsgefäß, auf die man nach dem Einfüllen der Komponenten einen Stickstoffdruck von etwa 1 bis 2 bar gibt, erhitzt anschließend die gewünschte Zeit auf die Reaktionstemperatur und entspannt nach dem Abkühlen.

Zur weiteren Reinigung kann man das erhaltene 1,7-Dibromperylen-3,4,9,10-tetracarbonsäurediimid IV in einem halogenierten Kohlenwasserstoff wie Methylenchlorid, Chloroform oder Tetrachlorethan lösen, über Kieselgel filtrieren und das Filtrat zur Trockene einengen. Das so gereinigte Diimid IV hat üblicherweise eine Reinheit von > 98 % und kann direkt für die Folgeumsetzungen verwendet werden.

Schritt b) des erfindungsgemäßen Herstellungsverfahrens, die Umsetzung des 1,7-Dibromperylen-3,4,9,10-tetracarbonsäurediimids IV mit dem 1-Alkin V, wird in Gegenwart eines aprotischen Lösungsmittels, eines Palladiumkomplexes als Katalysator, eines Kupfersalzes als Cokatalysator und einer Base vorgenommen.

Als Lösungsmittel eignen sich dabei lineare und cyclische aliphatische Ether mit bis zu 10 C-Atomen wie Diethylether, Di-n-propylether, Di-n-butylether, 1,2-Dimethoxyethan, 1,2-Diethoxyethan, Dioxan und insbesondere Tetrahydrofuran.

Die Menge an Lösungsmittel ist an sich nicht kritisch, üblicherweise kommen 30 bis 100 kg, vorzugsweise 40 bis 60 kg, Lösungsmittel je kg Diimid IV zum Einsatz.

Die zugesetzte Base dient gleichzeitig als Cosolvens. Geeignet sind hierfür vor allem mit den Ethern mischbare, organische Stickstoffbasen mit einem Schmelzpunkt unterhalb Raumtemperatur und einem Siedepunkt oberhalb der Reaktionstemperatur.

Bevorzugte Basen sind aliphatische Amine mit bis zu 15 C-Atomen, insbesondere tertiäre Amine wie Triethylamin, Tri-n-propylamin und Tri-n-butylamin und cycloaliphatische Amine wie insbesondere Piperidin.

Üblicherweise werden 0,2 bis 1,5 kg, bevorzugt 0,8 bis 1,2 kg, Base je kg Lösungsmittel zugesetzt.

Als Katalysator finden Palladiumkomplexe Verwendung, die in Verbindung mit Kupfer(I)salzen als Cokatalysator eingesetzt werden.

Beispiele für geeignete Palladiumkomplexe sind Tetrakis(tris-o-tolylphosphin)palladium(0), [1,2-Bis(diphenylphosphino)ethan]palladium(II)chlorid, [1,1'-Bis(diphenylphosphino)ferrocen]palladium(II)chlorid, Bis(triethylphosphin)palladium(II)chlorid, Bis(tricyclohexylphosphin)palladium(II)chlorid, Bis(triphenylphosphin)palladium(II)acetat, (2,2'-Bipyridyl)palladium(II)chlorid und insbesondere Tetrakis(triphenylphosphin)palladium(0), Bis(triphenylphosphin)palladium(II)chlorid, Bis(acetonitril)palladium(II)chlorid und Bis(benzonitril)palladium(II)chlorid.

Beispiele für besonders geeignete Kupfer(I)salze sind Kupfer(I)iodid und Kupfer(I)bromid.

In der Regel werden 2 bis 15 mol-%, vorzugsweise 5 bis 10 mol-%, Palladiumkomplex und im allgemeinen 2 bis 20 mol-%, bevorzugt 7 bis 12 mol-%, Kupfer(I)salz, jeweils bezogen auf das Diimid IV, eingesetzt.

Die Reaktionstemperatur beträgt in Schritt b) üblicherweise 20 bis 140°C, vor allem 40 bis 90°C.

Je nach dem eingesetzten Alkin kann die Umsetzung bei Normaldruck oder bei einem Überdruck von üblicherweise bis zu 50 bar durchgeführt werden. Die Arbeitsweise unter Druck ist erforderlich beim Einsatz flüchtiger Alkine wie Acetylen.

Bei der Herstellung symmetrischer Perylimide VI (VI') liegt das Molverhältnis der Ausgangsverbindungen Diimid IV und Alkin V in der Regel bei 1:2 bis 1:5, vorzugsweise bei 1:2 bis 1:4.

Bei der Herstellung unsymmetrischer Perylimide VI (VI"), bei der zunächst nur eines der beiden Bromatome im Perylengerüst durch die Reaktion mit einem Alkin Va ersetzt wird und anschließend das zweite Bromatom durch die Umsetzung mit einem unterschiedlichen Alkin Vb ersetzt wird, beträgt das Molverhältnis von Diimid IV zu Alkin V im allgemeinen jeweils 1:1 bis 1:2, bevorzugt 1:1 bis 1:1,5.

Üblicherweise ist die Umsetzung zu den symmetrischen Perylimiden VI' nach 1 bis 15 h, vor allem 2 bis 10 h, beendet. Dagegen dauert die Herstellung des monosubstituierten Monobromperylimids als Zwischenstufe bei der Umsetzung zu den unsymmetrischen Perylimiden VI" in der Regel nur 15 bis 60 min, insbesondere 15 bis 30 min. Für die weitere Umsetzung zum disubstituierten Perylimid VI" gelten die für die symmetrischen Perylimide VI' genannten Reaktionszeiten.

In Abhängigkeit von den gewählten Reaktionsbedingungen können in Schritt b) des erfindungsgemäßen Herstellungsverfahrens Perylimide VI hergestellt werden, die als Brückenglied L 1,2-Ethinylen- oder 1,2-Ethenylenreste enthalten.

Zur Herstellung ungesättigter Brückenglieder enthaltender Perylimide VI empfiehlt es sich, unter Schutzgas (z.B. Argon oder Stickstoff) zu arbeiten. Beträgt die Reaktionszeit über 4 h und/ oder liegt die Reaktionstemperatur über 100°C, so wird die acetylenische Bindung direkt zur ethylenischen Bindung reduziert.

Ethylenreste L enthaltende Perylimide VI können durch Nachrühren des Reaktionsgemisches in einer Wasserstoffatmosphäre erhalten werden. Man kann jedoch auch anschließend eine Reduktion der ungesättigten Bindung z.B. mit Wasserstoff unter Palladium/Aktivkohle-Katalyse vornehmen, wobei wie für derartige Reduktionen üblich vorgegangen werden kann (vgl. Larock, Comprehensive Organic Transformations, VCH Publishers New York, 1989, Seite 6 bis 17; March, Advanced Organic Chemistry, John Wiley and Sons New York, 4th Edition 1992, Seite 775 bis 777; Journal of Organic Chemistry, Band 45, Seite 4926 bis 4931 (1980)).

Verfahrenstechnisch geht man in Schritt b) zur Herstellung der symmetrischen Perylimide VI' zweckmäßigerweise wie folgt vor:

Man legt eine gerührte Lösung bzw. Suspension des Diimids IV in einer Mischung aus Lösungsmittel und Base (beide möglichst wasserfrei) vor, sättigt die Suspension mit Stickstoff durch mehrfaches Entgasen und Belüften mit trockenem Stickstoff, trägt im Stickstoff-Gegenstrom das Kupfer(I)salz, den Palladiumkomplex und das 1-Alkin V ein (flüchtige Alkine wie Acetylen werden abgewogen in die geschlossene Apparatur eingegast) und erhitzt die Reaktionsmischung für die gewünschte Zeit (≤ 4 h oder > 4 h) auf die gewünschte Reaktionstemperatur. Gewünschtenfalls wird nun mit Wasserstoff begast und weitere 4 bis 8 h bei der Reaktionstemperatur gerührt. Anschließend (gegebenenfalls nach vorherigem Entspannen) trägt man die Reaktionsmischung direkt, d.h. ohne vorheriges Abkühlen, unter starkem Rühren in das ca. dreifache Volumen einer Mischung aus etwa gleichen Gewichtsteilen konzentrierter Salzsäure und Eis ein, filtriert das Rohprodukt ab, wäscht mit halbkonzentrierter Salzsäure bis zum farblosen Ablauf und anschließend mit Wasser bis zum neutralen Ablauf und trocknet.

Zur weiteren Reinigung kann man das erhaltene Perylimid VI aus geeigneten Lösungsmitteln wie, z.B. halogenierten Kohlenwasserstoffen wie Methylenchlorid, Chloroform oder Tetrachlorethan oder cyclischen Ethern wie Dioxan oder Tetrahydrofuran umkristallisieren oder über eine kurze Kieselgelsäule chromatographieren, wobei je nach der Funktionalität des Restes R die obengenannten Lösungsmittel oder auch deren Mischungen als Eluens verwendet werden können.

Zur Herstellung der unsymmetrischen Perylimide VI" geht man verfahrenstechnisch zweckmäßigerweise wie bereits für die symmetrischen Perylimide VI' beschrieben vor, setzt jedoch zunächst nur die reduzierte Menge an 1-Alkin Va zu und erhitzt für 15 bis 30 min auf die gewünschte Reaktionstemperatur. Man kann dann das monosubstituierte Monobromperylimid VI wie oben beschrieben zuerst isolieren oder auch direkt mit dem zweiten 1-Alkin Vb durch 2- bis 4-stündiges oder längeres Rühren bei der gewünschten Reaktionstemperatur umsetzen.

Die Aufarbeitung des Reaktionsgemisches und die weitere Reinigung des Rohproduktes kann selbstverständlich wie oben für die symmetrischen Perylimide VI' beschrieben erfolgen.

Die so gereinigten Perylimide VI haben üblicherweise eine Reinheit von > 98 % und können direkt für die Folgeumsetzung eingesetzt werden. Sie können jedoch auch selbst bereits als wertvolle Pigmente und Farbstoffe dienen.

Schritt c) des erfindungsgemäßen Herstellungsverfahrens, die Verseifung des Perylimids VI zum Dianhydrid I, wird (gegebenenfalls nach vorheriger Reduktion des Perylimids) in Gegenwart eines polaren protischen Lösungsmittels und einer Base vorgenommen.

Als polare protische Lösungsmittel kommen dabei insbesondere C₁-C₁₀-Alkanole wie Ethanol, Propanol, n-Butanol, tert.-Butanol, 2-Methyl-2-butanol, n-Hexanol, n-Decanol und vorzugsweise Isopropanol in Frage. Zweckmäßigerweise wird zur Beschleunigung der Verseifungsreaktion noch Wasser, im allgemeinen 0,1 bis 0,2 mol je mmol Perylimid VI, zugesetzt.

Die Menge an Lösungsmittel ist an sich nicht kritisch, üblicherweise werden 50 bis 200 kg, bevorzugt 60 bis 80 kg, je kg Perylimid VI eingesetzt.

Als Base eignen sich vor allem Alkalimetallhydroxide wie Natriumhydroxid und Kaliumhydroxid, die in der Regel in Mengen von 4 bis 10 kg, vorzugsweise 5 bis 7 kg, je kg Perylimid VI zum Einsatz kommen.

Die Reaktionstemperatur beträgt in Schritt c) üblicherweise 20 bis 140°C, insbesondere 40 bis 90°C.

Im allgemeinen ist die Verseifung in 3 bis 10 h, vor allem 4 bis 6 h, beendet.

Verfahrenstechnisch geht man in Schritt c) des erfindungsgemäßen Herstellungsverfahrens zweckmäßigerweise wie folgt vor:

Man erhitzt eine Mischung von Perylimid VI, Lösungsmittel und Base für 4 bis 6 h auf die gewünschte Reaktionstemperatur, filtriert das nach Abkühlen auf Raumtemperatur ausgefallene Rohprodukt ab und wäscht es mit einem Alkohol wie Isopropanol oder Propanol bis zum farblosen Ablauf. Zur weiteren Reinigung trägt man das erhaltene Dianhydrid I zweckmäßigerweise in die 30- bis 100-fache Menge verdünnter anorganischer Säure, z.B. 5 bis 10 gew.-%iger Salzsäure, ein, kocht kurz auf, filtriert nach dem Abkühlen ab, wäscht mit Wasser neutral und trocknet.

Mit Hilfe des erfindungsgemäßen Herstellungsverfahrens können die 1,7-disubstituierten Perylen-3,4,9,10-tetracarbonsäuredianhydride I auf verfahrenstechnisch einfache, wirtschaftliche Weise in hohen Reinheiten (im allgemeinen > 95 %) und guten Ausbeuten erhalten werden.

Die erfindungsgemäßen Dianhydride I eignen sich vorteilhaft zur Pigmentierung von Druckfarben, Anstrichmitteln, insbesondere Tagesleuchtfarben und Kunststoffen, als Laserfarbstoffe und als Vorstufen für die Herstellung von Fluoreszenzfarbstoffen, polymeren Farbmitteln, Pigmenten und Pigmentadditiven.

Auch die erfindungsgemäßen Perylimide VI können vorteilhaft als Pigmente und Farbstoffe zum Einfärben von hochmolekularen organischen und von anorganischen Materialien, als Laserfarbstoffe und organische Materialien für Elektrolumineszenzanwendungen verwendet werden.

### Beispiele

### A) Herstellung von 1,7-Dibromperylen-3,4,9,10-tetracarbonsäuredianhydrid (II)

### Beispiel 1

Eine Mischung von 292,5 g (0,75 mol) Perylen-3,4,9,10-tetracarbonsäuredianhydrid (Wertgehalt > 98 %) und 4420 g 100 gew.-%iger Schwefelsäure wurde nach 12stündigem Rühren bei Raumtemperatur und anschließender Zugabe von 7 g Iod auf 85°C erhitzt. Dann wurden 262,5 g (1,64 mol) Brom in 8 h zugetropft.

Nach Abkühlen auf Raumtemperatur und Verdrängen des überschüssigen Broms durch Stickstoff wurde die Schwefelsäurekonzentration des Reaktionsgemisches durch portionsweise Zugabe von insgesamt 670 g Wasser in 1 h auf 86 Gew.-% erniedrigt. Nach dem Abkühlen des sich dabei wieder auf 85°C erhitzenden Reaktionsgemisches auf Raumtemperatur wurde das ausgefallene Produkt über eine G4-Glasfritte abfiltriert, mit 3 kg 86 gew.-%iger Schwefelsäure gewaschen, dann in 5 l Wasser aufgerührt, erneut abfiltriert, neutral gewaschen und bei 120°C im Vakuum getrocknet.

Es wurden 370 g II in Form eines leuchtendroten, feinkristallinen Pulvers mit einem Schmelzpunkt > 360°C und einem Wertgehalt von > 98 % erhalten, was einer Ausbeute von 90 % entspricht.

Analytische Daten:
Elementaranalyse (Gew.-% ber./gef.):
C: 52,4/52,1; H: 1,1/1,1; O: 17,45/17,4; Br: 29,1/29,4;
IR (KBr): ν = 1782 + 1770 (s, C=O), 1735 + 1723 (s, C=O) cm⁻¹;
UV/VIS (H₂SO₄): λₘₐₓ (ε) = 408 (10309), 520 (29410), 554 (43141) nm.

### B) Herstellung von 1,7-Dibromperylen-3,4,9,10-tetracarbonsäurediimiden IV

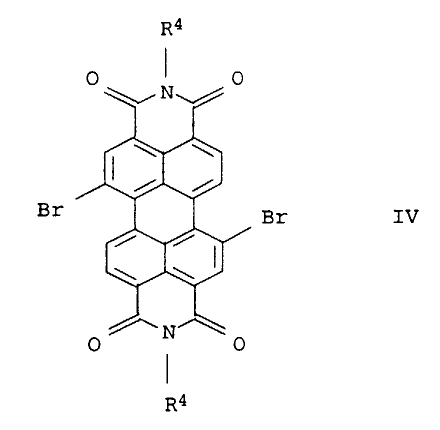

### Beispiele 2 und 3

Zu einer Mischung von 69,9 g (127 mmol) 1,7-Dibromperylen-3,4,9,10-tetracarbonsäuredianhydrid (II) (Beispiel 1) in 900 ml N-Methylpyrrolidon wurden unter Rühren zunächst a g des Imidierungskatalysators K und dann portionsweise insgesamt 381 mmol des primären Amins R⁴-NH₂ (III) zugegeben. Anschließend wurde das Reaktionsgemisch unter Stickstoff auf die Reaktionstemperatur T°C erhitzt und 6 h bei dieser Temperatur gerührt.

Nach dem Abkühlen auf Raumtemperatur wurde das ausgefallene Reaktionsprodukt abfiltriert, mit insgesamt 2 l Methanol gewaschen und bei 100°C im Vakuum getrocknet.

Weitere Einzelheiten zu diesen Versuchen sowie deren Ergebnisse sind in Tabelle 1 zusammengefaßt.

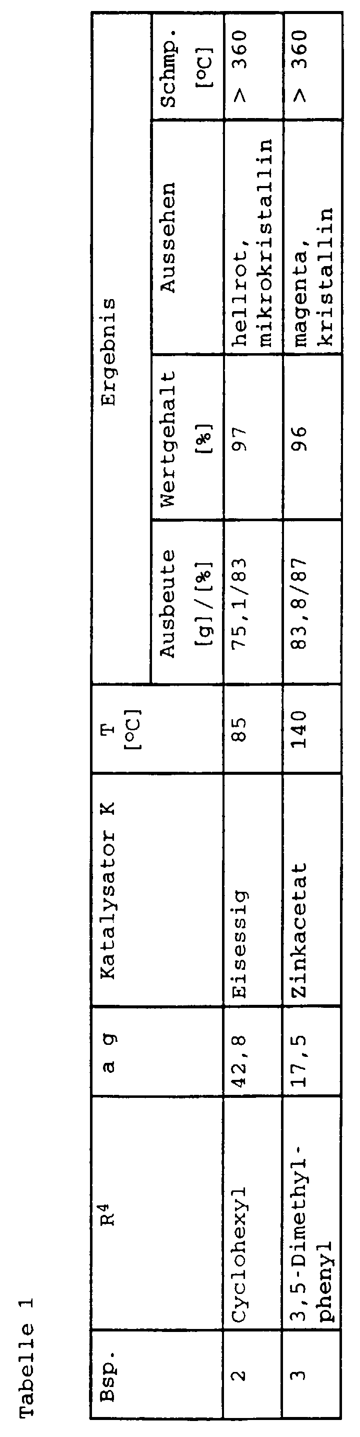

### Beispiel 2a

Zur weiteren Reinigung wurden 65,3 g des N,N'-Dicyclohexyl-1,7-dibromperylen-3,4,9,10-tetracarbonsäurediimids (IVa) aus Beispiel 2 1 h in 800 ml Methylenchlorid gerührt und anschließend über eine zu 2/3 mit Kieselgel (0,063-0,2 mm Korngröße) gefüllte 11-G4-Glasfritte filtriert (Methylenchlorid als Eluens).

Aus dem Filtrat wurden nach Verdampfen des Methylenchlorids im Vakuum 58 g IVa als orangerotes, kristallines Pulver mit einem Wertgehalt > 99 % erhalten.

Analytische Daten:
Elementaranalyse (Gew.-% ber./gef.):
C: 60,7/60,6; H: 4,0/4,0; N: 3,9/3,9; O: 9,0/9,0;
Br: 22,4/22,3;
IR (KBr): ν = 1698 (s, C=O), 1655 (s, C=O) cm⁻¹;
UV/VIS (CHCl₃): λₘₐₓ (ε) = 491 (33962), 526 (50478) nm.

### Beispiel 3a

Analog Beispiel 2a wurden 80 g des N,N'-Bis(3',5'-dimethylphenyl)-1,7-dibromperylen-3,4,9,10-tetracarbonsäurediimids (IVb) aus Beispiel 3 unter Verwendung von heißem Tetrachlorethan als Eluens gereinigt.

Es wurden 75 g IVb als leuchtendrotes, kristallines Pulver mit einem Wertgehalt > 98 % erhalten.

Analytische Daten:
Elementaranalyse (Gew.-% ber./gef.):
C: 63,5/63,3; H: 3,2/3,2; N: 3,7/3,7; O: 8,5/8,6;
Br: 21,1/21,2;
Masse (FD): m/z = 754 (M⁺, 100 %);
IR (KBr): ν = 1696 (s, C=O), 1653 (s, C=O) cm⁻¹;
UV/VIS (CHCl₃): λₘₐₓ (ε) = 485 (29214), 532 (45100) nm.

### C) Herstellung von 1,7-disubstituierten Perylen-3,4,9,10-tetracarbonsäurediimiden VI

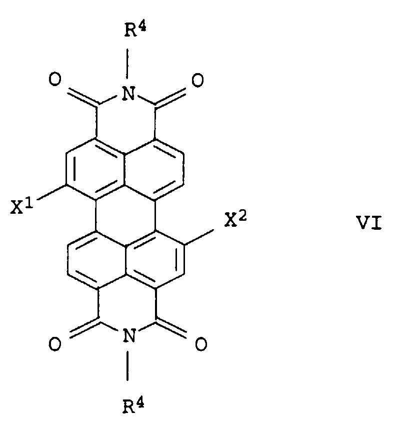

### Beispiele 4 bis 10

2,45 mmol des gereinigten 1,7-Dibromperylen-3,4,9,10-tetracarbonsäurediimids (IV) aus Beispiel 2a bzw. 3a wurden im Stickstoffgegenstrom unter Rühren in eine Mischung aus 100 ml absolutem Tetrahydrofuran und 100 ml frisch destilliertem Piperidin (Beispiele 4 bis 6 und 9 bis 10) bzw. 100 ml Triethylamin (Beispiele 7 und 8) eingetragen, nacheinander mit 45 mg (0,23 mmol) Kupfer(I)iodid, 225 mg (0,19 mmol) Tetrakis(triphenylphosphin)palladium(0) und b mg (b' mmol) des 1-Alkins V versetzt und unter Stickstoff t h auf 80°C (Beispiele 4 bis 6 und 9 bis 10) bzw. 60°C (Beispiele 7 und 8) erhitzt.

Die erhaltene schwarzviolett (Beispiel 9: dunkelgrün) gefärbte Reaktionsmischung wurde nach dem Abkühlen auf Raumtemperatur unter Rühren in 600 ml halbkonzentrierte Salzsäure eingetragen. Das ausgefallene Reaktionsprodukt wurde zunächst mit 400 ml halbkonzentrierter Salzsäure gewaschen, anschließend mit Wasser neutral gewaschen und bei 100°C im Vakuum getrocknet.

Das Rohprodukt wurde in 80 ml Methylenchlorid gelöst und über Kieselgel (0,063-0,2 mm Korngröße) mit Methylenchlorid als Eluens chromatographiert (Säule 330x100 mm).

Weitere Einzelheiten zu diesen Versuchen sowie deren Ergebnisse sind in Tabelle 2 zusammengestellt.

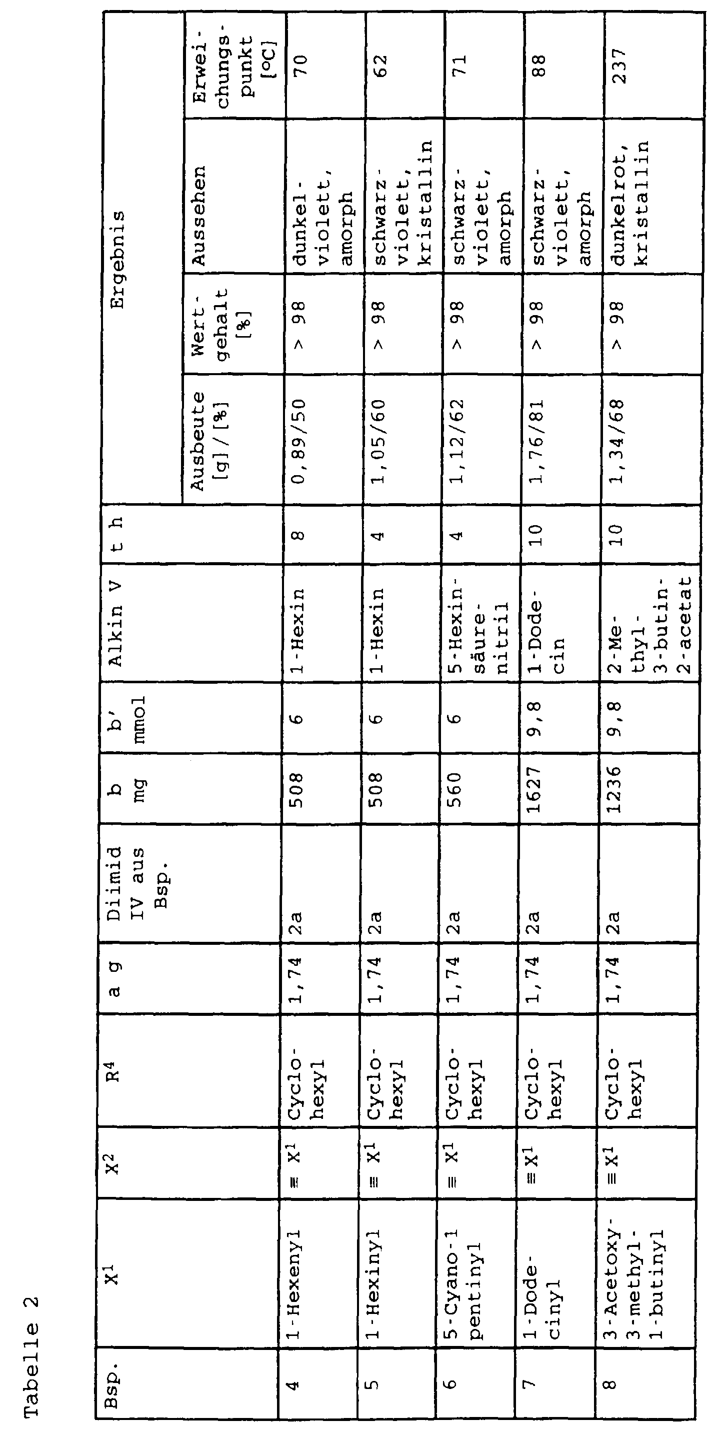

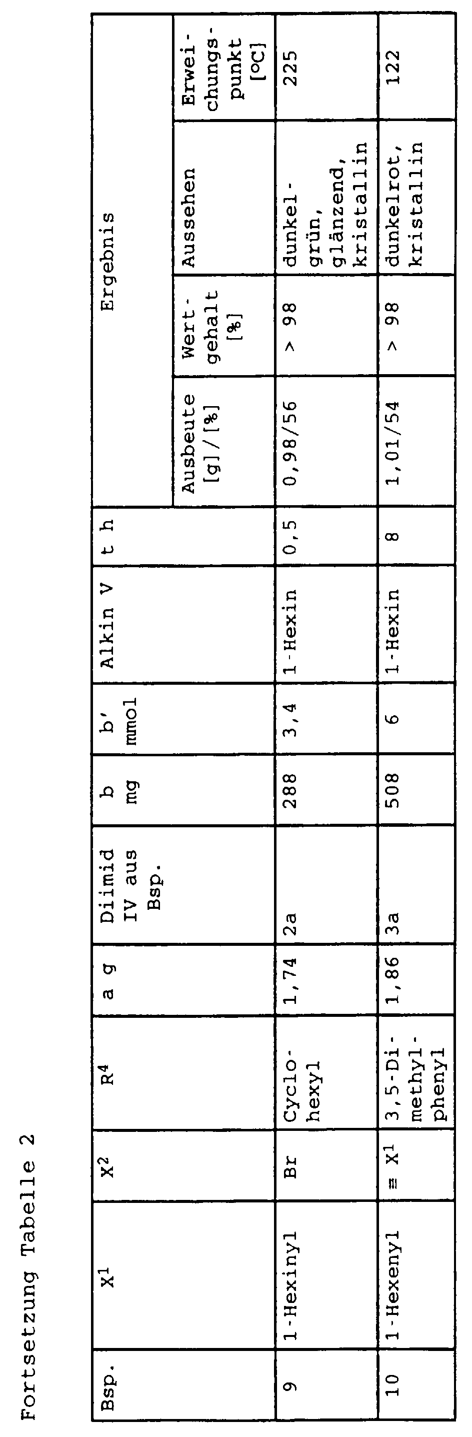

Analytische Daten zu Beispiel 4:
Elementaranalyse (Gew.-% ber./gef.):
C: 80,2/80,0; H: 7,0/7,1; N: 3,9/3,8; O: 8,9/9,1;
Masse (FD): m/z = 718 (M⁺, 100 %);
IR (KBr): ν = 1696 (s, C=O), 1650 (s, C=O) cm⁻¹;
UV/VIS (CHCl₃): λₘₐₓ (ε) = 286 (22906), 342 (12569), 398 (10544), 442 (9515), 469 (17094), 540 (12179) nm.

Analytische Daten zu Beispiel 5:
Elementaranalyse (Gew.-% ber./gef.):
C: 80,6/79,8; H: 6,5/6,7; N: 3,9/3,8; O: 8,95/9,6;
Masse (FD): m/z = 714 (M⁺, 100 %) ;
IR (KBr): ν = 2210 (w, C≡C), 1695 (s, C=O), 1649 (s, C=O) cm⁻¹;
UV/VIS (CHCl₃): λₘₐₓ (ε) = 293 (31542), 403 (15601), 441 (7321), 471 (11359), 538 (26011) nm.

Analytische Daten zu Beispiel 6:
Elementaranalyse (Gew.-% ber./gef.):
C: 78,2/77,7; H: 5,5/5,6; N: 7,6/7,5; O: 8,7/9,1;
Masse (FD): m/z = 736 (M⁺, 100 %);
IR (KBr): ν = 2238 (m, C≡N), 1698 (s, C=O), 1653 (s, C=O) cm⁻¹;
UV/VIS (CHCl₃): λₘₐₓ (ε) = 292 (30108), 403 (15831), 442 (6913), 472 (12003), 540 (24631) nm.

Analytische Daten zu Beispiel 7:
Elementaranalyse (Gew.-% ber./gef.):
C: 81,6/81,5; H: 8,0/8,0; N: 3,2/3,2; O: 7,25/7,3;
Masse (FD): m/z = 882 (M⁺, 100 %);
IR (KBr): ν = 2206 (w, C=C), 1695 (s, C=O), 1650 (s, C=O) cm⁻¹;
UV/VIS (CHCl₃): λₘₐₓ (ε) = 285 (17642), 294 (19311), 471 (6729), 505 (12936), 543 (21103) nm.

Analytische Daten zu Beispiel 8:
Elementaranalyse (Gew.-% ber./gef.):
C: 74,8/74,6; H: 5,75/5,8; N: 3,5/3,5; O: 15,95/16,1;
Masse (FD): m/z = 802 (M⁺, 100 %);
IR (KBr): ν = 2209 (w, C=C), 1741 (s, C=O, Ester), 1695 (s, C=O), 1650 (S, C=O) cm⁻¹;
UV/VIS (CHCl₃): λₘₐₓ (ε) = 288 (19733), 293 (21004), 473 (6610), 514 (14263), 565 (25317) nm.

Analytische Daten zu Beispiel 9 :
Elementaranalyse (Gew.-% ber./gef.):
C: 70,7/71,3; H: 5,2/5,3; N: 3,9/4,1;
O: 9,0/8,7; Br: 11,2/10,6;
Masse (FD): m/z = 712/714 (M⁺, ⁷⁹Br/⁸¹Br, 100 %);
IR (KBr): ν = 1690 (s, C=O), 1652 (s, C=O) cm⁻¹;
UV/VIS (CHCl₃): λₘₐₓ (ε) = 448 (11950), 629 (15300) nm.

Analytische Daten zu Beispiel 10:
Elementaranalyse (Gew.-% ber./gef.):
C: 81,9/81,7; H: 6,0/6,1; N: 3,7/3,7; O: 8,4/8,5:
Masse (FD): m/z = 762 (M⁺, 100 %);
IR (KBr): ν = 1694 (s, C=O), 1649 (s, C=O) cm⁻¹;
UV/VIS (CHCl₃): λₘₐₓ (ε) = 290 (25703), 339 (8112), 401 (10291), 458 (15388), 559 (23900) nm.

### Beispiel 11

0,8 g (1,12 mmol) des N,N'-Dicyclohexyl-1-hexinyl-7-bromperylen-3,4,9,10-tetracarbonsäurediimids aus Beispiel 9 wurden analog zu der oben aufgeführten Vorschrift in 4 h mit 0,14 g (1,5 mmol) 5-Hexinsäurenitril umgesetzt.

Nach säulenchromatographischer Reinigung wurden 0,52 g N,N'-Dicyclohexyl-1-hexinyl-7-(5'-cyanopentinyl)-perylen-3,4,9,10-tetracarbonsäurediimid (X¹: 1-Hexinyl; X²: 5-Cyano-1-pentinyl) in Form eines schwarzvioletten, mikrokristallinen Pulvers mit einem Erweichungspunkt von 83°C und einem Wertgehalt von 98 % erhalten, was einer Ausbeute von 64 % entspricht.

Analytische Daten:
Elementaranalyse (Gew.-% ber./gef.):
C: 79,4/79,2; H: 6,0/6,1; N: 5,8/5,8; O: 8,8/8,9;
Masse (FD): m/z = 725 (M⁺, 100 %);
IR (KBr): ν = 2236 (m, C≡N), 1695 (s, C=O), 1650 (s, C=O) cm⁻¹;
UV/VIS (CHCl₃): λₘₐₓ (ε) = 293 (31542), 403 (15601), 441 (7321), 471 (11359), 538 (26011) nm.

### Beispiel 12

1,0 g (1,4 mmol) des N,N'-Dicyclohexyl-1,7-dihexenylperylen-3,4,9,10-tetracarbonsäurediimis aus Beispiel 4 wurden in 150 ml Methanol gelöst, mit 0,5 g eines handelsüblichen Hydrierungskatalysators (5 % Palladium auf Aktivkohle) versetzt und bei Raumtemperatur und einem Wasserstoffdruck von 1 bar hydriert.

Nach Abfiltrieren des Katalysators und Abdestillieren des Lösungsmittels wurden 0,92 g N,N'-Dicyclohexyl-1,7-dihexylperylen-3,4,9,10-tetracarbonsäurediimid (X¹ = X² = Hexyl) in Form eines schwarzroten, amorphen Pulvers mit einem Erweichungspunkt von 112°C und einem Wertgehalt von 98 % erhalten, was einer Ausbeute von 91 % entspricht.

Analytische Daten:
Elementaranalyse (Gew.-% ber./gef.):
C: 79,75/79,7; H: 7,55/7,5; N: 3,85/3,8; O: 8,85/8,9;
Masse (FD) : m/z = 722 (M⁺, 100 %);
IR (KBr): ν = 1695 (s, C=O), 1650 (s, C=O) cm⁻¹;
UV/VIS (CHCl₃): λₘₐₓ (ε) = 278 (19712), 339 (11823), 398 (7210), 463 (8132), 510 (20101), 554 (29300) nm.

### D) Herstellung von 1,7-disubstituierten Perylen-3,4,9,10-tetracarbonsäuredianhydriden I

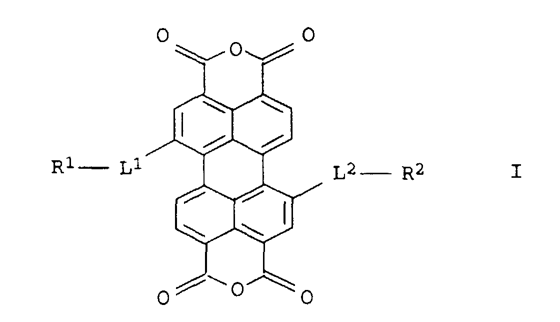

### Beispiel 13

Eine Mischung von 10 g des N,N'-Dicyclohexyl-1,7-dihexenylperylen-3,4,9,10-tetracarbonsäurediimids aus Beispiel 4, 1 l Isopropanol, 65 g Kaliumhydroxid und 26 g Wasser wurde 5 h unter Rückfluß erhitzt.

Nach dem Abkühlen auf Raumtemperatur wurde das ausgefallene Reaktionsprodukt abfiltriert, mit Isopropanol bis zum farblosen Ablauf gewaschen, dann unter Rühren in 1 l 10 gew.-%ige Salzsäure eingetragen und kurz zum Sieden erhitzt. Nach Abkühlen auf Raumtemperatur wurde das Produkt erneut abfiltriert, mit Wasser neutral gewaschen und bei 100°C im Vakuum getrocknet.

Es wurden 6,8 g 1,7-Dihexenylperylen-3,4,9,10-tetracarbonsäuredianhydrid (L¹ = L² = 1,2-Ethylen, R¹ = R² = n-Butyl) als dunkelrotes, amorphes Pulver mit einem Schmelzpunkt > 360°C und einem Wertgehalt > 98 % (Bestimmung durch UV/VIS-Spektroskopie und halbquantitative Dünnschichtchromatographie an Kieselgel mit Trichloressigsäure/Toluol als mobile Phase) erhalten, was einer Ausbeute von 88 % entspricht.

### Beispiel 14

Analog Beispiel 13 wurden 10 g des N,N'-Bis(3',5'-dimethylphenyl)-1,7-dihexenylperylen-3,4,9,10-tetracarbonsäurediimids aus Beispiel 10 umgesetzt.

Es wurden 6,2 g 1,7-Dihexenylperylen-3,4,9,10-tetracarbonsäuredianhydrid der gleichen Qualität wie in Beispiel 13 erhalten, was einer Ausbeute von 85 % entspricht.

Analytische Daten zu Beispiel 13 und 14:
Elementaranalyse (Gew.-% ber./gef.):
C: 77,7/77,6; H: 5,1/5,1; O: 17,2/17,3;
IR (KBr): ν = 1769 (s, C=O), 1722 (s, C=O) cm⁻¹;
UV/VIS (CHCl₃): λₘₐₓ (ε) = 399 (12732), 532 (46311) nm.

## Patentansprüche

1. 1,7-Disubstituierte Perylen-3,4,9,10-tetracarbonsäuredianhydride der allgemeinen Formel I und Perylen-3,4,9,10-tetracarbonsäuren der allgemeinen Formel Ia 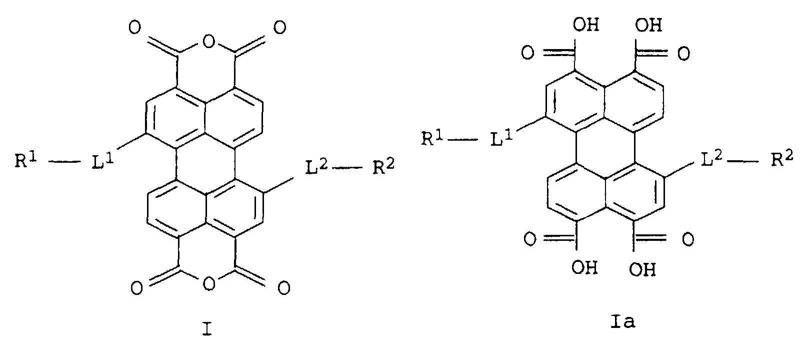 in der die Variablen folgende Bedeutung haben:
L¹, L² unabhängig voneinander 1,2-Ethylen, 1,2-Ethenylen und 1,2-Ethinylen;
R¹, R² unabhängig voneinander Wasserstoff oder C₁-C₃₀-Alkyl, dessen Kohlenstoffkette durch eine oder mehrere Gruppierungen -O-, -S-, -NR³-, -CO- und/oder -SO₂- unterbrochen sein kann und/oder das durch -COOR³, -SO₃R³, Hydroxy, Cyano, C₁-C₆-Alkoxy, C₅-C₈-Cycloalkyl, Aryl oder einen über ein Stickstoffatom gebundenen, 5- bis 7-gliedrigen heterocyclischen Rest, der weitere Heteroatome enthalten kann und/oder aromatisch sein kann, ein- oder mehrfach substituiert sein kann, wobei R³ für Wasserstoff oder C₁-C₆-Alkyl steht.

2. Perylen-3,4,9,10-tetracarbonsäuredianhydride der Formel I und Perylen-3,4,9,10-tetracarbonsäuren der Formel Ia nach Anspruch 1, in der
L¹, L² gleich sind und 1,2-Ethenylen oder 1,2-Ethinylen und
R¹, R² unabhängig voneinander Wasserstoff oder C₁-C₁₈-Alkyl, das durch -COOR³, Hydroxy oder Cyano substituiert sein kann,
bedeuten.

3. Verfahren zur Herstellung von symmetrischen, 1,7-disubstituierten Perylen-3,4,9,10-tetracarbonsäuredianhydriden der Formel I bzw. den Perylen-3,4,9,10-tetracarbonsäuren der Formel Ia gemäß Anspruch 1 oder 2, in der R¹ und R² sowie L¹ und L² jeweils gleich sind, dadurch gekennzeichnet, daß man
a) 1,7-Dibromperylen-3,4,9,10-tetracarbonsäuredianhydrid (II) bzw. 1,7-Dibromperylen-3,4,9,10-tetracarbonsäure (IIa) in Gegenwart eines polaren aprotischen Lösungsmittels und gegebenenfalls eines Imidierungskatalysators mit einem primären Amin der allgemeinen Formel III
R⁴ - NH₂ III
in der R⁴ C₄-C₃₀-Alkyl, dessen Kohlenstoffkette durch eine oder mehrere Gruppierungen -O-, -S- oder -CO- unterbrochen sein kann, C₅-C₈-Cycloalkyl oder Aryl, das durch C₁-C₆-Alkyl oder C₁-C₆-Alkoxy ein- oder mehrfach substituiert sein kann, bedeutet, umsetzt,
b) die in Schritt a) gebildeten 1,7-Dibromperylen-3,4,9,10-tetracarbonsäurediimide der allgemeinen Formel IV 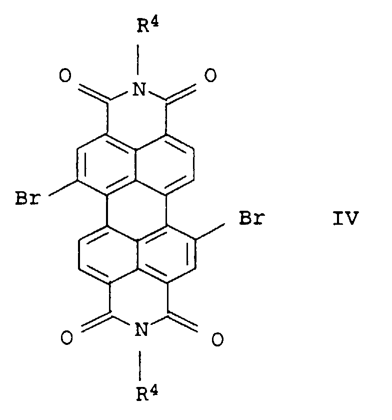 in Gegenwart eines aprotischen Lösungsmittels, eines Palladiumkomplexes als Katalysator, eines Kupfersalzes als Cokatalysator und einer Base mit einem 1-Alkin der allgemeinen Formel V
H - C ≡ C - R¹ V
in einem Molverhältnis von 1:2 bis 1:4 umsetzt und
c) die in Schritt b) gebildeten symmetrischen, 1,7-disubstituierten Perylen-3,4,9,10-tetracarbonsäurediimide der allgemeinen Formel VI' 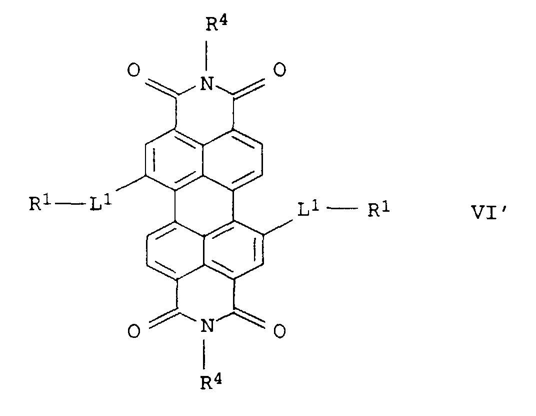 gewünschtenfalls nach zusätzlicher Reduktion der ungesättigten Bindungen in L¹ in Gegenwart eines polaren protischen Lösungsmittels und einer Base zu den symmetrischen, 1,7-disubstituierten Perylen-3,4,9,10-tetracarbonsäuredianhydriden (I) bzw. den Perylen-3,4,9,10-tetracarbonsäuren (Ia) verseift.

4. Verfahren zur Herstellung von unsymmetrischen, 1,7-disubstituierten Perylen-3,4,9,10-tetracabonsäuredianhydriden der Formel I bzw. den Perylen-3,4,9,10-tetracarbonsäuren der Formel Ia gemäß Anspruch 1 oder 2, in der R¹ und R² verschieden und L¹ und L² gleich oder verschieden sind, dadurch gekennzeichnet, daß man
a) 1,7-Dibromperylen-3,4,9,10-tetracarbonsäuredianhydrid (II) bzw. 1,7-Dibromperylen-3,4,9,10-tetracarbonsäure (IIa) in Gegenwart eines polaren aprotischen Lösungsmittels und gegebenenfalls eines Imidierungskatalysators mit einem primären Amin der allgemeinen Formel III
R⁴ - NH₂ III
in der R⁴ C₄-C₃₀-Alkyl, dessen Kohlenstoffkette durch eine oder mehrere Gruppierungen -O-, -S- oder -CO- unterbrochen sein kann, C₅-C₈-Cycloalkyl oder Aryl, das durch C₁-C₆-Alkyl oder C₁-C₆-Alkoxy ein- oder mehrfach substituiert sein kann, bedeutet, umsetzt,
b) die in Schritt a) gebildeten l,7-Dibromperylen-3,4,9,10-tetracarbonsäurediimide der allgemeinen Formel IV 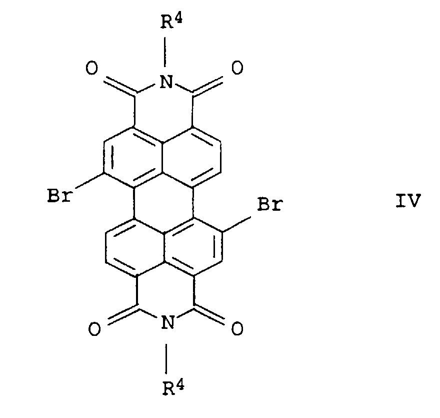 in Gegenwart eines aprotischen Lösungsmittels, eines Palladiumkomplexes als Katalysator, eines Kupfersalzes als Cokatalysator und einer Base zunächst mit einem 1-Alkin der allgemeinen Formel Va
H - C ≡ C - R1 Va
und anschließend mit einem anderen 1-Alkin der allgemeinen Formel Vb
H - C ≡ C - R² Vb
jeweils in einem Molverhältnis von 1:1 bis 1:2 umsetzt und
c) die in Schritt b) gebildeten unsymmetrischen, 1,7-disubstituierten Perylen-3,4,9,10-tetracarbonsäurediimide der allgemeinen Formel VI" 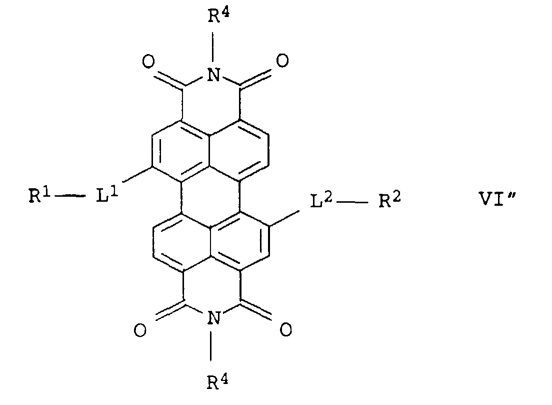 gewünschtenfalls nach zusätzlicher Reduktion der ungesättigen Bindungen in L¹ und L² in Gegenwart eines polaren protischen Lösungsmittels und einer Base zu den unsymmetrischen, 1,7-disubstituierten Perylen-3,4,9,10-tetracarbonsäuredianhydriden (I) bzw. den Perylen-3,4,9,10-tetracarbonsäuren (Ia) verseift.

5. 1,7-Disubstituierte Perylen-3,4,9,10-tetracarbonsäurediimide der allgemeinen Formel VI 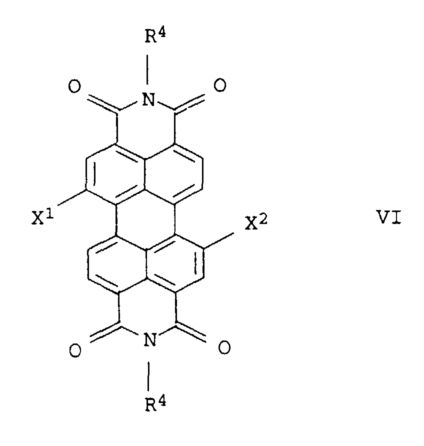 in der die Variablen folgende Bedeutung haben:
X¹ Brom oder einen Rest der Formel -L-R, wobei
L für 1,2-Ethylen, 1,2-Ethenylen oder 1,2-Ethinylen steht und
R Wasserstoff oder C₁-C₃₀-Alkyl bedeutet, dessen Kohlenstoffkette durch eine oder mehrere Gruppierungen -O-, -S-, -NR³-, -CO- und/oder -SO₂- unterbrochen sein kann und/oder das durch -COOR³, -SO₃R³, Hydroxy, Cyano, C₁-C₆-Alkoxy, C₅-C₈-Cycloalkyl, Aryl oder einen über ein Stickstoffatom gebundenen, 5- bis 7-gliedrigen heterocyclischen Rest, der weitere Heteroatome enthalten kann und/oder aromatisch sein kann, ein- oder mehrfach substituiert sein kann, wobei R³ für Wasserstoff oder C₁-C₆-Alkyl steht;
X² Brom oder einen Rest der Formel -L-R;
R⁴ C₄-C₃₀-Alkyl, dessen Kohlenstoffkette durch eine oder mehrere Gruppierungen -O-, -S- oder -CO- unterbrochen sein kann, C₅-C₈-Cycloalkyl oder Aryl, das durch C₁-C₆-Alkyl oder C₁-C₆-Alkoxy ein- oder mehrfach substituiert sein kann.

6. Perylen-3,4,9,10-tetracarbonsäurediimide der allgemeinen Formel VI nach Anspruch 5, in der die Variablen folgende Bedeutung haben:
X¹ und X² Brom oder gleiche Reste der Formel -L-R, wobei
L für 1,2-Ethenylen oder 1,2-Ethinylen und
R für Wasserstoff, C₁-C₁₈-Alkyl, das durch -COOR³, Hydroxy, Cyano, C₁-C₆-Alkoxy, C₅-C₈-Cycloalkyl oder Aryl ein- oder mehrfach substituiert sein kann, steht;
R⁴ C₅-C₈-Cycloalkyl oder Phenyl, das in den meta- und/oder para-Positionen durch C₁-C₆-Alkyl oder C₁-C₆-Alkoxy substituiert sein kann.

7. Verfahren zur Herstellung von symmetrischen, 1,7-disubstituierten Perylen-3,4,9,10-tetracarbonsäurediimiden der Formel VI gemäß Anspruch 5 oder 6, in der X¹ und X² gleiche Reste -L-R bedeuten, dadurch gekennzeichnet, daß man
a) 1,7-Dibromperylen-3,4,9,10-tetracarbonsäuredianhydrid (II) bzw. 1,7-Dibromperylen-3,4,9,10-tetracarbonsäure (IIa) in Gegenwart eines polaren aprotischen Lösungsmittels und gegebenenfalls eines Imidierungskatalysators mit einem primären Amin der allgemeinen Formel III
R⁴ - NH₂ III
umsetzt und
b) die in Schritt a) gebildeten 1,7-Dibromperylen-3,4,9,10-tetracarbonsäurediimide der allgemeinen Formel IV 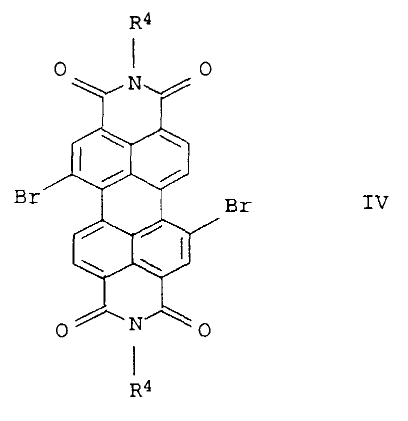 in Gegenwart eines aprotischen Lösungsmittels, eines Palladiumkomplexes als Katalysator, eines Kupfersalzes als Cokatalysator und einer Base mit einem 1-Alkin der allgemeinen Formel V
H - C ≡ C - R¹ V
in einem Molverhältnis von 1:2 bis 1:4 umsetzt.

8. Verfahren zur Herstellung von unsymmetrischen, 1,7-disubstituierten Perylen-3,4,9,10-tetracarbonsäurediimiden der Formel VI gemäß Anspruch 5 oder 6, in der X¹ und X² verschiedene Reste -L-R bedeuten, dadurch gekennzeichnet, daß man
a) 1,7-Dibromperylen-3,4,9,10-tetracarbonsäuredianhydrid (II) bzw. 1,7-Dibromperylen-3,4,9,10-tetracarbonsäure (IIa) in Gegenwart eines polaren aprotischen Lösungsmittels und gegebenenfalls eines Imidierungskatalysators mit einem primären Amin der allgemeinen Formel III
R⁴ - NH₂ III
umsetzt und
b) die in Schritt a) gebildeten 1,7-Dibromperylen-3,4,9,10-tetracarbonsäurediimide der allgemeinen Formel IV 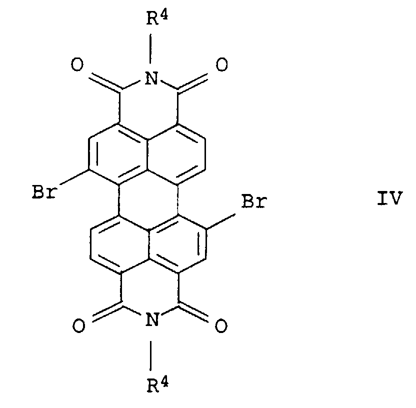 in Gegenwart eines aprotischen Lösungsmittels, eines Palladiumkomplexes als Katalysator, eines Kupfersalzes als Cokatalysator und einer Base zunächst mit einem 1-Alkin der allgemeinen Formel Va
H - C ≡ C - R¹ Va
und anschließend mit einem anderen 1-Alkin der allgemeinen Formel Vb
H - C ≡ C - R² Vb
jeweils in einem Molverhältnis von 1:1 bis 1:2 umsetzt.

9. Verfahren zur Herstellung von 1,7-Dibromperylen-3,4,9,10-tetracarbonsäurediimiden der allgemeinen Formel IV 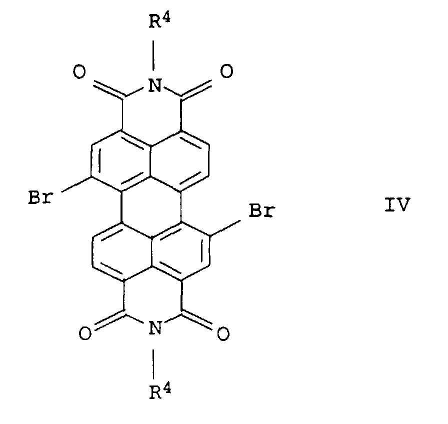 in der R⁴ C₄-C₃₀-Alkyl, dessen Kohlenstoffkette durch eine oder mehrere Gruppierungen -O-, -S- oder -CO- unterbrochen sein kann, C₅-C₈-Cycloalkyl oder Aryl, das durch C₁-C₆-Alkyl oder C₁-C₆-Alkoxy ein- oder mehrfach substituiert sein kann, bedeutet, dadurch gekennzeichnet, daß man 1,7-Dibromperylen-3,4,9,10-tetracarbonsäuredianhydrid (II) bzw. 1,7-Dibromperylen-3,4,9,10-tetracarbonsäure (IIa) in Gegenwart eines polaren aprotischen Lösungsmittels und gegebenenfalls eines Imidierungskatalysators mit einem primären Amin der allgemeinen Formel III
R⁴ - NH₂ III
umsetzt.

10. Verwendung von 1,7-disubstituierten Perylen-3,4,9,10-tetracarbonsäuredianhydriden der Formel I oder Perylen-3,4,9,10-tetracarbonsäuren der Formel Ia gemäß Anspruch 1 oder 2 als Pigmente, Laserfarbstoffe und Vorstufen für die Herstellung von Fluoreszenzfarbstoffen, polymeren Farbmitteln, Pigmenten und Pigmentadditiven.

11. Verwendung von 1,7-disubstituierten Perylen-3,4,9,10-tetracarbonsäurediimiden der Formel VI gemäß Anspruch 5 oder 6 als Pigmente und Farbstoffe zum Einfärben von hochmolekularen organischen und von anorganischen Materialien, als Laserfarbstoffe und organische Materialien für Elektrolumineszenzanwendungen.

## Claims

1. A 1,7-disubstituted perylene-3,4,9,10-tetracarboxylic dianhydride of the general formula I or perylene-3,4,9,10-tetracarboxylic acid of the general formula Ia 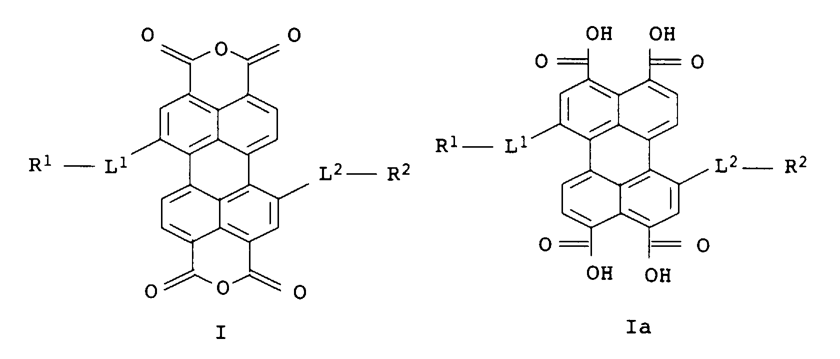 where
L¹, L² independently of one another are 1,2-ethylene, 1,2-ethenylene and 1,2-ethynylene;
R¹, R² independently of one another are hydrogen or C₁-C₃₀-alkyl, whose carbon chain can be interrupted by one or more groups -O-, -S-, -NR³-, -CO- and/or -SO₂- and/or which can be substituted one or more times by -COOR³, -SO₃R³, hydroxyl, cyano, C₁-C₆-alkoxy, C₅-C₈-cycloalkyl or aryl or by a 5- to 7-membered heterocyclic radical which is attached via a nitrogen atom and can include further heteroatoms and/or can be aromatic, R³ being hydrogen or C₁-C₆-alkyl.

2. A compound as claimed in claim 1, where
L¹, L² are identical and are 1,2-ethenylene or 1,2-ethynylene and
R¹, R² independently of one another are hydrogen or C₁-C₁₈-alkyl which can be substituted by -COOR³, hydroxyl or cyano.

3. A process for preparing a symmetrical compound of the formula I or a compound of the formula Ia, as claimed in claim l or 2, where R¹ and R² and L¹ and L² are each identical, which comprises
a) reacting 1,7-dibromoperylene-3,4,9,10-tetracarboxylic dianhydride (II) or 1,7-dibromoperylene-3,4,9,10-tetracarboxylic acid (IIa) in the presence of a polar aprotic solvent and in the presence or absence of an imidation catalyst with a primary amine of the general formula III
R⁴ - NH₂ III
where R⁴ is C₄-C₃₀-alkyl whose carbon chain can be interrupted by one or more groups -O-, -S- or -CO-, or is C₅-C₈-cycloalkyl or aryl which can be substituted one or more times by C₁-C₆-alkyl or C₁-C₆-alkoxy,
b) reacting the 1,7-dibromoperylene-3,4,9,10-tetracarboxylic diimide formed in step a), of the general formula IV 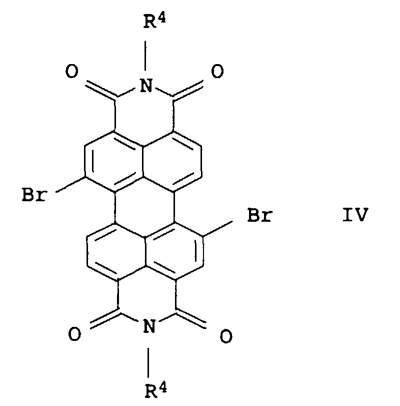 in the presence of an aprotic solvent, a palladium complex as catalyst, a copper salt as cocatalyst and a base with a 1-alkyne of the general formula V
H - C ≡ C - R¹ V
in a molar ratio of from 1:2 to 1:4, and
c) hydrolyzing the symmetrical, 1,7-disubstituted perylene-3,4,9,10-tetracarboxylic diimide, formed in step b), of the general formula VI' 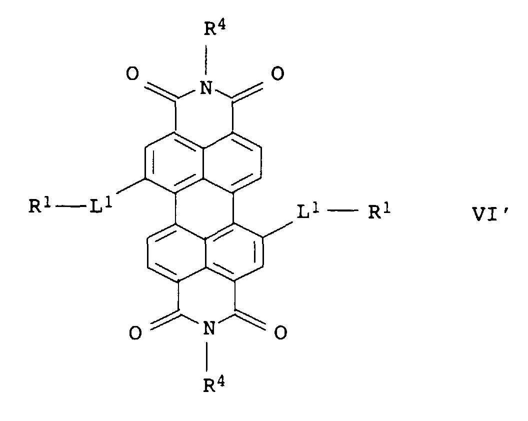 after additional reduction of the unsaturated bonds in L¹, if desired, in the presence of a polar protic solvent and a base to form the symmetric 1,7-disubstituted perylene-3,4,9,10-tetracarboxylic dianhydride (I) or the perylene-3,4,9,10-tetracarboxylic acid (Ia).

4. A process for preparing an asymmetric compound of the formula I or compound of the formula Ia, as claimed in claim 1 or 2, in which R¹ and R² are different and L¹ and L² are identical or different, which comprises
a) reacting 1,7-dibromoperylene-3,4,9,10-tetracarboxylic dianhydride (II) or 1,7-dibromoperylene-3,4,9,10-tetracarboxylic acid (IIa) in the presence of a polar aprotic solvent and in the presence or absence of an imidation catalyst with a primary amine of the general formula III
R⁴ - NH₂ III
where R⁴ is C₄-C₃₀-alkyl whose carbon chain can be interrupted by one or more groups -O-, -S- or -CO-, or is C₅-C₈-cycloalkyl or aryl which can be substituted one or more times by C₁-C₆-alkyl or C₁-C₆-alkoxy,
b) reacting the 1,7-dibromoperylene-3,4,9,10-tetracarboxylic diimide formed in step a), of the general formula IV 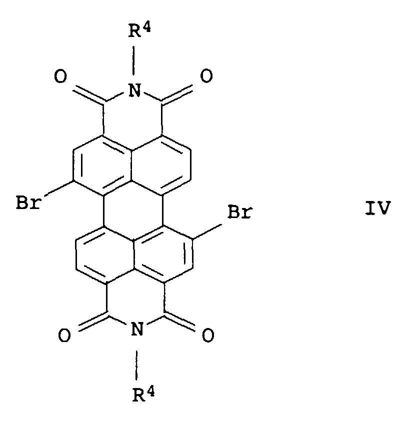 in the presence of an aprotic solvent, a palladium complex as catalyst, a copper salt as cocatalyst and a base, first with a 1-alkyne of the general formula Va
H - C ≡ C - R¹ Va
and then with a different 1-alkyne of the general formula Vb
H - C ≡ C - R² Vb
in each case in a molar ratio of from 1:1 to 1:2, and
c) hydrolyzing the asymmetric, 1,7-disubstituted perylene-3,4,9,10-tetracarboxylic diimide, formed in step b), of the general formula VI" 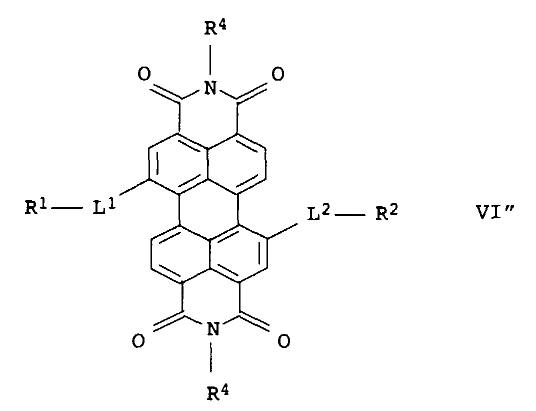 after additional reduction of the unsaturated bonds in L¹ and L², if desired, in the presence of a polar protic solvent and a base to form the asymmetric 1,7-disubstituted perylene-3,4,9,10-tetracarboxylic dianhydride (I) or the perylene-3,4,9,10-tetracarboxylic acid (Ia).

5. A 1,7-disubstituted perylene-3,4,9,10-tetracarboxylic diimide of the general formula VI 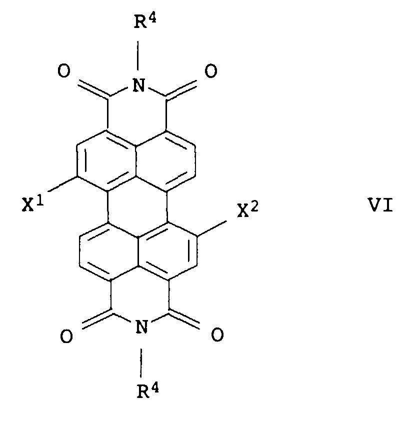 where
X¹ is bromine or is -L-R, where
L is 1,2-ethylene, 1,2-ethenylene or 1,2-ethynylene and
R is hydrogen or C₁-C₃₀-alkyl whose carbon chain can be interrupted by one or more groups -O-, -S-, -NR³-, -CO- and/or -SO₂- and/or which can be substituted one or more times by -COOR³, -SO₃R³, hydroxyl, cyano, C₁-C₆-alkoxy, C₅-C₈-cycloalkyl or aryl or by a 5- to 7-membered heterocyclic radical which is attached via a nitrogen atom and can include further heteroatoms and/or can be aromatic, R³ being hydrogen or C₁-C₆-alkyl;
X² is bromine or -L-R;
R⁴ is C₄-C₃₀-alkyl whose carbon chain can be interrupted by one or more groups -O-, -S- or -CO-, or is C₅-C₈-cycloalkyl or aryl which can be substituted one or more times by C₁-C₆-alkyl or C₁-C₆-alkoxy.

6. A compound as claimed in claim 5, where:
X¹ and X² are bromine or identical radicals -L-R, where
L is 1,2-ethenylene or 1,2-ethynylene and
R is hydrogen or C₁-C₁₈-alkyl which can be substituted one or more times by -COOR³, hydroxyl, cyano, C₁-C₆-alkoxy, C₅-C₈-cycloalkyl or aryl;
R⁴ is C₅-C₈-cycloalkyl or phenyl which can be substituted in the meta and/or para positions by C₁-C₆-alkyl or C₁-C₆-alkoxy.

7. A process for preparing a symmetrical compound of the formula VI as claimed in claim 5 or 6, where X¹ and X² are identical radicals -L-R, which comprises
a) reacting 1,7-dibromoperylene-3,4,9,10-tetracarboxylic dianhydride (II) or 1,7-dibromoperylene-3,4,9,10-tetracarboxylic acid (IIa) in the presence of a polar aprotic solvent and in the presence or absence of an imidation catalyst with a primary amine of the general formula III
R⁴ - NH₂ III
and
b) reacting the 1,7-dibromoperylene-3,4,9,10-tetracarboxylic diimide formed in step a), of the general formula IV 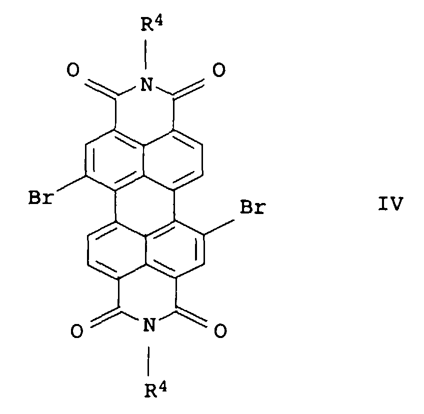 in the presence of an aprotic solvent, a palladium complex as catalyst, a copper salt as cocatalyst and a base with a 1-alkyne of the general formula V
H - C ≡ C - R¹ V
in a molar ratio of from 1:2 to 1:4.

8. A process for preparing an asymmetrical compound of the formula VI as claimed in claim 5 or 6, where X¹ and X² are different radicals -L-R, which comprises
a) reacting 1,7-dibromoperylene-3,4,9,10-tetracarboxylic dianhydride (II) or 1,7-dibromoperylene-3,4,9,10-tetracarboxylic acid (IIa) in the presence of a polar aprotic solvent and in the presence or absence of an imidation catalyst with a primary amine of the general formula III
R⁴ - NH₂ III
and
b) reacting the 1,7-dibromoperylene-3,4,9,10-tetracarboxylic diimide formed in step a), of the general formula IV 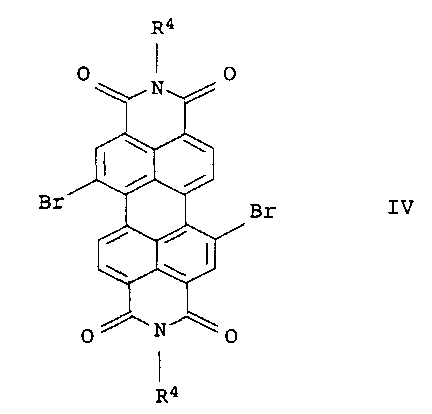 in the presence of an aprotic solvent, a palladium complex as catalyst, a copper salt as cocatalyst and a base, first with a 1-alkyne of the general formula Va
H - C ≡ C - R¹ Va
and then with a different 1-alkyne of the general formula Vb
H - C ≡ C - R² Vb
in each case in a molar ratio of from 1:1 to 1:2.

9. A process for preparing a 1,7-dibromoperylene-3,4,9,10-tetracarboxylic diimide of the general formula IV 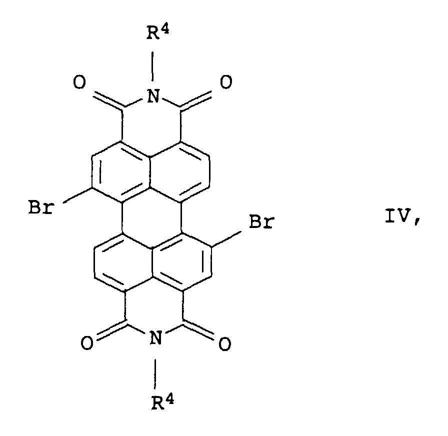 where R⁴ is C₄-C₃₀-alkyl whose carbon chain can be interrupted by one or more groups -O-, -S- or -CO-, or is C₅-C₈-cycloalkyl or aryl which can be substituted one or more times by C₁-C₆-alkyl or C₁-C₆-alkoxy, which comprises reacting 1,7-dibromoperylene-3,4,9,10-tetracarboxylic dianhydride (II) or, respectively, 1,7-dibromoperylene-3,4,9,10-tetracarboxylic acid (IIa) in the presence of a polar aprotic solvent and in the presence or absence of an imidation catalyst with a primary amine of the general formula III.
R⁴ - NH₂ III.

10. The use of a compound as claimed in claim 1 or 2 as a pigment, laser dye or precursor for preparing fluorescent dyes, polymeric colorants, pigments and pigment additives.

11. The use of a compound as claimed in claim 5 or 6 as a pigment or dye for coloring high molecular mass organic materials and inorganic materials, as a laser dye or as an organic material for electroluminescence applications.

## Revendications

1. Dianhydrides pérylène-3,4,9,10-tétracarboxyliques de formule générale I et acides pérylène-3,4,9,10-tétracarboxyliques de formule générale la, disubstitués en 1,7 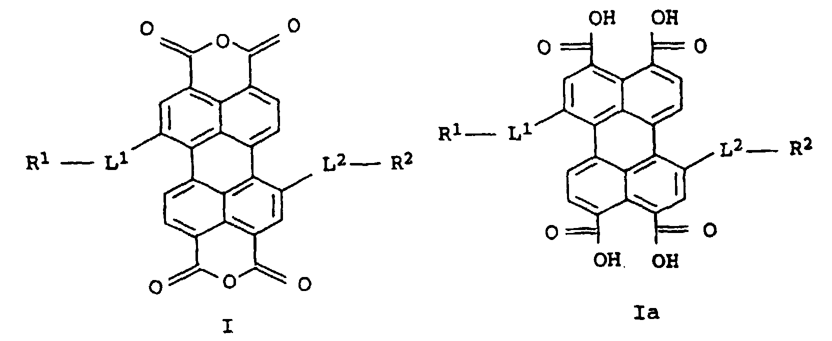 dans lesquelles les variables prennent la signification suivante :
L¹ L² représentent indépendamment l'un de l'autre un groupe 1,2-éthylène, 1,2-éthénylène et 1,2-éthynylène ;
R¹, R² représentent indépendamment l'un de l'autre un atome d'hydrogène ou un groupe alkyle en C₁ à C₃₀, dont la chaîne carbonée peut être interrompue par un ou plusieurs groupements -O-, -S-, -NR³-, -CO- et/ou -SO₂-, et/ou qui peut être mono- ou polysubstitué par un groupe -COOR³, -SO₃R³, hydroxy, cyano, alcoxy en C₁ à C₆, cycloalkyle en C₅ à C₈, aryle ou par un résidu hétérocyclique de 5 à 7 éléments liés par l'intermédiaire d'un atome d'azote, qui peut contenir d'autres hétéroatomes et/ou peut être aromatique, R³ représentant un atome d'hydrogène ou un groupe alkyle en C₁ à C₆.

2. Dianhydrides pérylène-3,4,9,10-tétracarboxyliques de formule I et acides pérylène-3,4,9,10-tétracarboxyliques de formule la selon la revendication 1, dans lesquelles
L¹, L² sont identiques et représentent un groupe 1,2-éthénylène ou 1,2-éthynylène et
R¹, R² représentent indépendamment l'un de l'autre un atome d'hydrogène ou un groupe alkyle en C₁ à C₁₈, qui peut être substitué par un groupe COOR³, hydroxy ou cyano.

3. Procédé pour produire des dianhydrides pérylène-3,4,9,10-tétracarboxyliques de formule I ou des acides pérylène-3,4,9,10-tétracarboxyliques de formule la, symétriques et disubstitués en 1,7 selon la revendication 1 ou 2, dans lesquelles R¹ et R² ainsi que L¹ et L² sont respectivement identiques, caractérisé en ce que,
a) l'on met à réagir le dianhydride 1,7-dibromopérylène-3,4,9,10-tétracarboxylique (II) ou l'acide 1,7-dibromopérylène-3,4,9,10-tétracarboxylique (IIa) en présence d'un solvant polaire aprotique et éventuellement d'un catalyseur d'imidation, avec une amine primaire de formule générale III
R⁴ - NH₂ III
dans laquelle R⁴ représente un groupe alkyle en C₄ à C₃₀, dont la chaîne carbonée peut être interrompue par un ou plusieurs groupements -O-, -S- ou -CO-, un groupe cycloalkyle en C₅ à C₈ ou aryle, qui peut être mono- ou polysubstitué par un groupe alkyle en C₁ à C₆ ou par un groupe alcoxy en C₁ à C₆,
b) l'on met à réagir les diimides 1,7-dibromopérylène-3,4,9,10-tétra-carboxyliques formés dans l'étape a) de formule générale IV 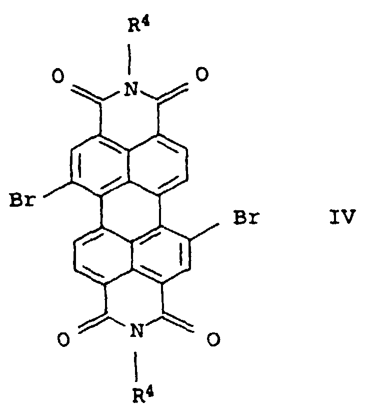 en présence d'un solvant aprotique, d'un complexe du palladium en tant que catalyseur, d'un sel de cuivre en tant que co-catalyseur et d'une base, avec un 1-alcyne de formule générale V
H-C≡C-R¹ V
dans un rapport molaire de 1:2 à 1:4 et
c) on saponifie les diimides pérylène-3,4,9,10-tétracarboxyliques disubstitués en 1,7, symétriques formés dans l'étape b) de formule générale VI' 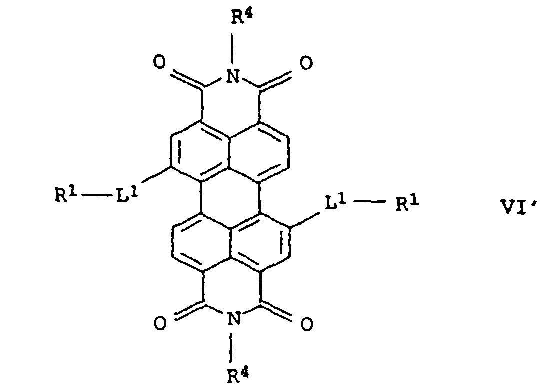 éventuellement après une réduction supplémentaire des liaisons insaturées dans L¹ en présence d'un solvant polaire protique et d'une base, en les dianhydrides pérylène-3,4,9,10-tétracarboxyliques symétriques (I) ou les acides pérylène-3,4,9,10-tétracarboxyliques (la) disubstitués en 1,7.

4. Procédé pour produire des dianhydrides pérylène-3,4,9,10-tétracarboxyliques de formule I ou des acides pérylène-3,4,9,10-tétracarboxyliques de formule la, disubstitués en 1,7 et non symétriques, selon la revendication 1 ou 2, dans lesquelles R¹ et R¹ sont différents et L¹ et L² sont identiques ou différents, caractérisé en ce que
a) l'on met à réagir le dianhydride 1,7-dibromopérylène-3,4,9,10-tétracarboxylique (II) ou l'acide 1,7-dibromopérylène-3,4,9,10-tétracarboxylique (IIa) en présence d'un solvant polaire aprotique et éventuellement d'un catalyseur d'imidation, avec une amine primaire de formule générale III
R⁴ - NH₂ III
dans laquelle R⁴ représente un groupe alkyle en C₄ à C₃₀, dont la chaîne carbonée peut être interrompue par un ou plusieurs groupements -O-, -S- ou -CO-, un groupe cycloalkyle en C₅ à C₈ ou aryle, qui peut être mono- ou polysubstitué par un groupe alkyle en C₁ à C₆ ou alcoxy en C₁ à C₆,
b) l'on met d'abord à réagir les diimides 1,7-dibromopérylène-3,4,9,10-tétracarboxyliques formés dans l'étape a) de formule générale IV 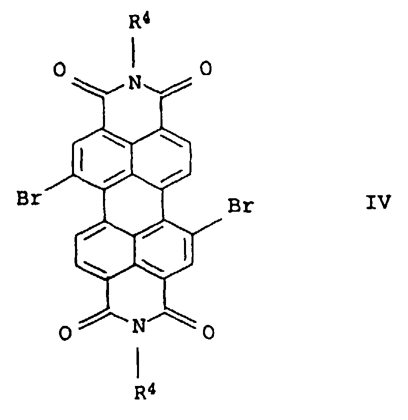 en présence d'un solvant aprotique, d'un complexe du palladium en tant que catalyseur, d'un sel de cuivre en tant que co-catalyseur et d'une base, avec un 1-alcyne de formule générale Va
H - C ≡ C - R¹ Va
et ensuite avec un autre 1-alcyne de formule générale Vb
H - C ≡ C - R² Vb
respectivement dans un rapport molaire de 1:1 à 1:2, et
c) l'on saponifie les diimides pérylène-3,4,9,10-tétracarboxyliques disubstitués en 1,7, non symétriques, formés dans l'étape b) de formule générale VI" 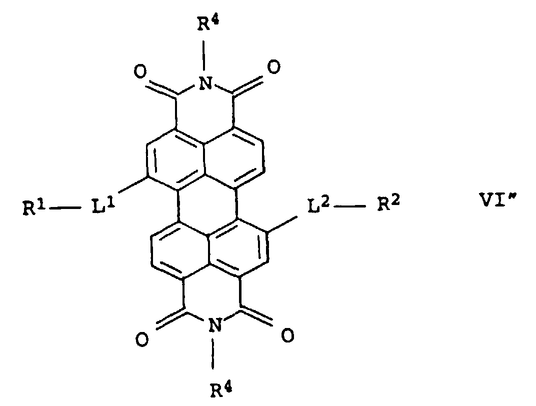 éventuellement après une une réduction supplémentaire des liaisons insaturées dans L¹ et L² en présence d'un solvant polaire protique et d'une base, en les dianhydrides pérylène-3,4,9,10-tétracarboxyliques (I) ou les acides pérylène-3,4,9,10-tétra-carboxyliques (la), disubstitués en 1,7, non symétriques.

5. Diimides pérylène-3,4,9,10-tétracarboxyliques disubstitués en 1,7 de formule générale VI 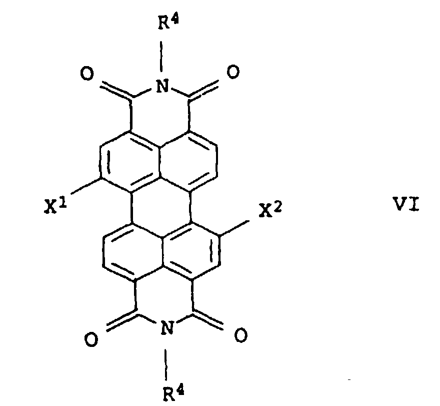 dans laquelle les variables prennent la signification suivante :
X¹ représente un atome de brome ou un résidu de formule -L-R, où
L représente un groupe 1,2-éthylène, 1,2-éthénylène ou 1,2-éthynylène et
R représente un atome d'hydrogène ou un groupe alkyle en C₁ à C₃₀, dont la chaîne carbonée peut être interrompue par un ou plusieurs groupements -O-, -S-, -NR³-, -CO- et/ou-SO₂- et/ou qui peut être mono- ou polysubstitué par un groupe -COOR³, -SO₃R³, hydroxy, cyano, alcoxy en C₁ à C₆, cycloalkyle en C₅ à C₈, aryle ou un résidu hétérocyclique de 5 à 7 éléments liés par l'intermédiaire d'un atome d'azote, qui peut contenir d'autres hétéroatomes et/ou être aromatique, R³ représentant un atome d'hydrogène ou un groupe alkyle en C₁ à C₆ ;
X² représente un atome de brome ou un résidu de formule -L-R ;
R⁴ représente un groupe alkyle en C₁ à C₃₀, dont la chaîne carbonée peut être interrompue par un ou plusieurs groupements -O-, -S-, et/ou -CO-, un groupe cycloalkyle en C₅ à C₈ ou aryle, qui peut être mono- ou polysubstitué par un groupe alkyle en C₁ à C₆ ou alcoxy en C₁ à C₆.

6. Diimides pérylène-3,4,9,10-tétracarboxyliques de formule générale VI selon la revendication 5, où les variables prennent la signification suivante:
X¹ et X² représentent un atome de brome ou le même résidu de formule -L-R, où
L représente un groupe 1,2-éthénylène ou 1,2-éthynylène et
R représente un atome d'hydrogène, un groupe alkyle en C₁ à C₁₈, qui peut être mono- ou polysubstitué par un groupe -COOR³, hydroxy, cyano, alcoxy en C₁ à C₆, cycloalkyle en C₅ à C₈ ou aryle ;
R⁴ représente un groupe cycloalkyle en C₅ à C₈ ou phényle, qui peut être substitué dans les positions méta et/ou para par un groupe alkyle en C₁ à C₆ ou alcoxy en C₁ à C₆.

7. Procédé pour produire les diimides pérylène-3,4,9,10-tétracarboxyliques disubstitués en 1,7, symétriques de formule VI selon les revendications 5 ou 6, dans lesquelles X¹ et X² représentent les mêmes résidus -L-R, caractérisés en ce que
a) l'on met à réagir le dianhydride 1,7-dibromopérylène-3,4,9,10-tétracarboxylique (II) ou l'acide 1,7-dibromopérylène-3,4,9,10-tétracarboxylique (lla) en présence d'un solvant polaire aprotique et éventuellement d'un catalyseur d'imidation, avec une amine primaire de formule générale III
R⁴ - NH₂ III
et
b) l'on met à réagir les diimides 1,7-dibromopérylène-3,4,9,10-tétracarboxyliques formés dans l'étape a) de formule générale IV 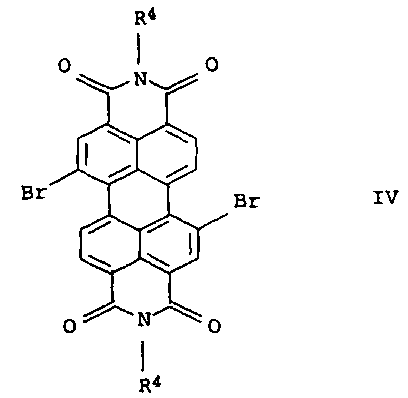 en présence d'un solvant aprotique, d'un complexe du palladium en tant que catalyseur, d'un sel de cuivre en tant que co-catalyseur et d'une base, avec un 1-alcyne de formule générale V
H - C ≡ C - R¹ V
dans un rapport molaire de 1:2 à 1:4.

8. Procédé pour produire les diimides pérylène-3,4,9,10-tétracarboxyliques disubstitués en 1,7, non symétriques de formule VI selon les revendications 5 ou 6, dans lesquelles X¹ et X² représentent des résidus -L-R différents, caractérisés en ce que
a) l'on met à réagir le dianhydride 1,7-dibromopérylène-3,4,9,10-tétracarboxylique (II) ou l'acide 1,7-dibromopérylène-3,4,9,10-tétracarboxylique (IIa) en présence d'un solvant polaire aprotique et éventuellement d'un catalyseur d'imidation, avec une amine primaire de formule générale III
R⁴ - NH₂ III
et
b) l'on met d'abord à réagir les diimides 1,7-dibromopérylène-3,4,9,10-tétracarboxyliques formés dans l'étape a) de formule générale IV 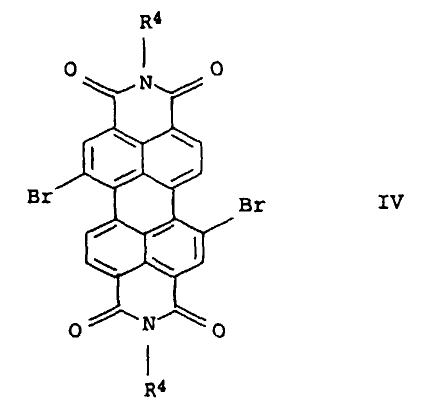 en présence d'un solvant aprotique, d'un complexe du palladium en tant que catalyseur, d'un sel de cuivre en tant que co-catalyseur et d'une base, avec un 1-alcyne de formule générale Va
H - C ≡ C - R¹ Va
et ensuite avec un autre 1-alcyne de formule générale Vb
H - C ≡ C - R² Vb
respectivement dans un rapport molaire de 1:1 à 1:2.

9. Procédé pour produire les diimides 1,7-dibromopérylène-3,4,9,10-tétracarboxyliques de formule générale IV 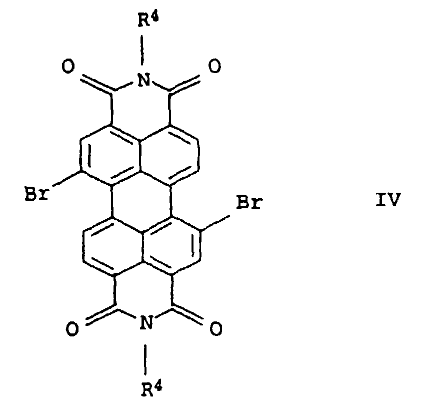 dans laquelle R⁴ représente un groupe alkyle en C₄ à C₃₀, dont la chaîne carbonée peut être interrompue par un ou plusieurs groupements -O-, -S-, -CO-, un groupe cycloalkyle en C₅ à C₈ ou aryle, qui peut être mono- ou polysubstitué par un groupe alkyle en C₁ à C₆ ou alcoxy en C₁ à C₆, caractérisé en ce que l'on met à réagir le dianhydride 1,7-dibromopérylène-3,4,9,10-tétra-carboxylique (II) ou l'acide 1,7-dibromopérylène-3,4,9,10-tétra-carboxylique (IIa) en présence d'un solvant polaire aprotique et éventuellement d'un catalyseur d'imidation, avec une amine primaire de formule générale III
R⁴ - NH₂ III

10. Utilisation des dianhydrides pérylène-3,4,9,10-tétracarboxyliques de formule I ou des acides pérylène-3,4,9,10-tétracarboxyliques de formule la, disubstitués en 1,7 selon la revendication 1 ou 2, en tant que pigments, colorants de laser et précurseurs pour la production de colorants à fluorescence, colorants polymères, pigments et additifs pour pigments.

11. Utilisation des diimides pérylène-3,4,9,10-tétracarboxyliques disubstitués en 1,7 de formule VI selon la revendication 5 ou 6, en tant que pigments et colorants pour colorer des matières organiques de masse moléculaire élevée et des matières inorganiques, en tant que colorants de laser et matières organiques pour des applications dans le domaine de l'électroluminescence.
